(19)

Europäisches
Patentamt
European
Patent Office
Office européen
des brevets

(11) **EP 3 812 008 A1**

(12) **EUROPEAN PATENT APPLICATION**

(43) Date of publication:
**28.04.2021 Bulletin 2021/17**

(51) Int Cl.:
***A61P 35/00*** *(2006.01)*  ***A61P 43/00*** *(2006.01)*
***C07K 16/28*** *(2006.01)*

(21) Application number: **19306377.3**

(22) Date of filing: **23.10.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(71) Applicant: **Gamamabs Pharma
31106 Toulouse (FR)**

(72) Inventors:
• **PROST, Jean-François
78000 VERSAILLES (FR)**

• **DUBREUIL, Olivier
31190 MAURESSAC (FR)**
• **BARRET, Jean-Marc
81100 CASTRES (FR)**
• **DEGOVE, Stéphane
93500 PANTIN (FR)**

(74) Representative: **Nony
11 rue Saint-Georges
75009 Paris (FR)**

(54) **AMH-COMPETITIVE ANTAGONIST ANTIBODY**

(57)     The present invention relates to a competitive antagonist antibody that specifically binds to AMHR-II, said antibody comprising (a) a heavy chain wherein the variable domain comprises a H-CDR1 having a sequence set forth as SEQ ID NO: 3; a H-CDR2 having a sequence set forth as SEQ ID NO: 4; and a H-CDR3 having a sequence set forth as SEQ ID NO: 5; and (b) a light chain wherein the variable domain comprises at least a CDR selected from the group consisting of a L-CDR1 having a sequence set forth as SEQ ID NO: 8, a L-CDR2 having a sequence set forth as SEQ ID NO: 9; and a L-CDR3 having a sequence set forth as SEQ ID NO: 10.

A nucleic acid sequence encoding said antibody, a vector comprising the nucleic acid sequence, a host cell comprising said nucleic acid sequence or vector, an immunoconjugate comprising the said antibody, a pharmaceutical composition comprising the said antibody or immunoconjugate, and the use as a drug of the said antibody, cell or composition is also considered in the present invention.

EP 3 812 008 A1

SEQUENCE LISTING

<110> GAMAMABS PHARMA

<120> AMH-COMPETITIVE ANTAGONIST ANTIBODY

<130> PR83978

<160> 12

<170> BiSSAP 1.3.6

<210> 1
<211> 451
<212> PRT
<213> Artificial Sequence

<220>
<223> amino acid sequence of heavy chain

<400> 1

```
Glu Val Gln Leu Val Glu Ser Gly Gly Ser Leu Val Lys Pro Gly Gly
1               5                   10                  15
Ser Leu Arg Leu Ser Cys Ala Ala Ser Gly Phe Thr Phe Ser Asn Asn
            20                  25                  30
Ala Met Asn Trp Val Arg Gln Ala Pro Gly Lys Gly Leu Glu Trp Ile
        35                  40                  45
Ser Tyr Ile Ser Gly Ser Ser Arg Tyr Ile Ser Tyr Ala Asp Phe Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Val Arg Ser Ser Asp Asp Tyr Phe Gly Gly Met Asp Val Trp Gly
            100                 105                 110
Arg Gly Thr Leu Val Thr Val Ser Ala Ser Thr Lys Gly Pro Ser
            115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Arg Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
        290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
```

```
                        325                    330                        335
        Glu Lys Thr Ile Ser Lys Ala Lys Gly Gln Pro Arg Glu Pro Gln Val
                    340                    345                    350
        Tyr Thr Leu Pro Pro Ser Arg Glu Glu Met Thr Lys Asn Gln Val Ser
                    355                    360                    365
        Leu Thr Cys Leu Val Lys Gly Phe Tyr Pro Ser Asp Ile Ala Val Glu
        370                    375                    380
        Trp Glu Ser Asn Gly Gln Pro Glu Asn Asn Tyr Lys Thr Thr Pro Pro
        385                    390                    395                    400
        Val Leu Asp Ser Asp Gly Ser Phe Phe Leu Tyr Ser Lys Leu Thr Val
                    405                    410                    415
        Asp Lys Ser Arg Trp Gln Gln Gly Asn Val Phe Ser Cys Ser Val Met
                    420                    425                    430
        His Glu Ala Leu His Asn His Tyr Thr Gln Lys Ser Leu Ser Leu Ser
                    435                    440                    445
        Pro Gly Lys
            450


        <210> 2
        <211> 121
        <212> PRT
        <213> Artificial Sequence


        <220>
        <223> amino acid sequence of heavy chain variable region


        <400> 2
        Glu Val Gln Leu Val Glu Ser Gly Gly Ser Leu Val Lys Pro Gly Gly
        1                   5                   10                  15
        Ser Leu Arg Leu Ser Cys Ala Ala Ser Gly Phe Thr Phe Ser Asn Asn
                    20                    25                    30
        Ala Met Asn Trp Val Arg Gln Ala Pro Gly Lys Gly Leu Glu Trp Ile
                    35                    40                    45
        Ser Tyr Ile Ser Gly Ser Ser Arg Tyr Ile Ser Tyr Ala Asp Phe Val
                50                    55                    60
        Lys Gly Arg Phe Thr Ile Ser Arg Asp Asn Ala Lys Asn Ser Leu Tyr
        65                    70                    75                    80
        Leu Gln Met Asn Ser Leu Arg Ala Glu Asp Thr Ala Val Tyr Tyr Cys
                        85                    90                    95
        Val Arg Ser Ser Asp Asp Tyr Phe Gly Gly Gly Met Asp Val Trp Gly
                    100                    105                    110
        Arg Gly Thr Leu Val Thr Val Ser Ser
                    115                    120


        <210> 3
        <211> 10
        <212> PRT
        <213> Artificial Sequence


        <220>
        <223> H-CDR1


        <400> 3
        Gly Phe Thr Phe Ser Asn Asn Ala Met Asn
        1                   5                       10


        <210> 4
        <211> 10
        <212> PRT
        <213> Artificial Sequence
```

```
<220>
<223> H-CDR2

<400> 4
Tyr Ile Ser Gly Ser Ser Arg Tyr Ile Ser
1               5                   10


<210> 5
<211> 12
<212> PRT
<213> Artificial Sequence


<220>
<223> H-CDR3

<400> 5
Ser Ser Asp Asp Tyr Phe Gly Gly Gly Met Asp Val
1               5                   10


<210> 6
<211> 216
<212> PRT
<213> Artificial Sequence


<220>
<223> amino acid sequence of the light chain

<400> 6
Gln Ser Val Leu Thr Gln Pro Ala Ser Val Ser Gly Ser Pro Gly Gln
1               5                   10                  15
Ser Ile Thr Ile Ser Cys Ala Gly Thr Ser Ser Asp Val Gly Gly Asp
                20              25                  30
Asn Asp Val Ser Trp Tyr Gln Gln His Pro Gly Lys Ala Pro Lys Leu
            35                  40                  45
Met Ile Tyr Tyr Asp Ser Tyr Arg Pro Ser Gly Val Ser Asn Arg Phe
        50                  55                  60
Ser Gly Ser Lys Ser Gly Asn Thr Ala Ser Leu Thr Ile Ser Gly Leu
65                  70                  75                      80
Gln Ala Glu Asp Glu Ala Asp Tyr Tyr Cys Ser Ser Ser Thr Tyr Tyr
                85                  90                  95
Ser Thr Arg Val Phe Gly Gly Gly Thr Lys Leu Thr Val Leu Gly Gln
            100                 105                 110
Pro Lys Ala Ala Pro Ser Val Thr Leu Phe Pro Pro Ser Ser Glu Glu
        115                 120                 125
Leu Gln Ala Asn Lys Ala Thr Leu Val Cys Leu Ile Ser Asp Phe Tyr
    130                 135                 140
Pro Gly Ala Val Thr Val Ala Trp Lys Ala Asp Ser Ser Pro Val Lys
145                 150                 155                 160
Ala Gly Val Glu Thr Thr Thr Pro Ser Lys Gln Ser Asn Asn Lys Tyr
                165                 170                 175
Ala Ala Ser Ser Tyr Leu Ser Leu Thr Pro Glu Gln Trp Lys Ser His
            180                 185                 190
Arg Ser Tyr Ser Cys Gln Val Thr His Glu Gly Ser Thr Val Glu Lys
        195                 200                 205
Thr Val Ala Pro Thr Glu Cys Ser
    210                 215


<210> 7
<211> 110
<212> PRT
```

<213> Artificial Sequence


<220>
<223> amino acid sequence of the light chain variable region

<400> 7
Gln Ser Val Leu Thr Gln Pro Ala Ser Val Ser Gly Ser Pro Gly Gln
1               5                   10                  15
Ser Ile Thr Ile Ser Cys Ala Gly Thr Ser Ser Asp Val Gly Gly Asp
            20                  25                  30
Asn Asp Val Ser Trp Tyr Gln Gln His Pro Gly Lys Ala Pro Lys Leu
        35                  40                  45
Met Ile Tyr Tyr Asp Ser Tyr Arg Pro Ser Gly Val Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Ser Thr Tyr Tyr
                85                  90                  95
Ser Thr Arg Val Phe Gly Gly Gly Thr Lys Leu Thr Val Leu
            100                 105                 110

<210> 8
<211> 14
<212> PRT
<213> Artificial Sequence


<220>
<223> L-CDR1

<400> 8
Ala Gly Thr Ser Ser Asp Val Gly Gly Asp Asn Asp Val Ser
1               5                   10

<210> 9
<211> 7
<212> PRT
<213> Artificial Sequence


<220>
<223> L-CDR2

<400> 9
Tyr Asp Ser Tyr Arg Pro Ser
1               5

<210> 10
<211> 10
<212> PRT
<213> Artificial Sequence


<220>
<223> L-CDR3

<400> 10
Ser Ser Ser Thr Tyr Tyr Ser Thr Arg Val
1               5                   10

<210> 11
<211> 556

```
<212> PRT
<213> Homo sapiens


<220>
<223> human AMHR-II amino acid sequence devoid of its signal peptide

<400> 11
Pro Pro Asn Arg Arg Thr Cys Val Phe Phe Glu Ala Pro Gly Val Arg
1               5                   10                  15
Gly Ser Thr Lys Thr Leu Gly Glu Leu Leu Asp Thr Gly Thr Glu Leu
            20                  25                  30
Pro Arg Ala Ile Arg Cys Leu Tyr Ser Arg Cys Cys Phe Gly Ile Trp
        35                  40                  45
Asn Leu Thr Gln Asp Arg Ala Gln Val Glu Met Gln Gly Cys Arg Asp
        50                  55                  60
Ser Asp Glu Pro Gly Cys Glu Ser Leu His Cys Asp Pro Ser Pro Arg
65                  70                  75                  80
Ala His Pro Ser Pro Gly Ser Thr Leu Phe Thr Cys Ser Cys Gly Thr
                85                  90                  95
Asp Phe Cys Asn Ala Asn Tyr Ser His Leu Pro Pro Pro Gly Ser Pro
            100                 105                 110
Gly Thr Pro Gly Ser Gln Gly Pro Gln Ala Ala Pro Gly Glu Ser Ile
        115                 120                 125
Trp Met Ala Leu Val Leu Leu Gly Leu Phe Leu Leu Leu Leu Leu Leu
        130                 135                 140
Leu Gly Ser Ile Ile Leu Ala Leu Leu Gln Arg Lys Asn Tyr Arg Val
145                 150                 155                 160
Arg Gly Glu Pro Val Pro Glu Pro Arg Pro Asp Ser Gly Arg Asp Trp
                165                 170                 175
Ser Val Glu Leu Gln Glu Leu Pro Glu Leu Cys Phe Ser Gln Val Ile
            180                 185                 190
Arg Glu Gly Gly His Ala Val Val Trp Ala Gly Gln Leu Gln Gly Lys
        195                 200                 205
Leu Val Ala Ile Lys Ala Phe Pro Pro Arg Ser Val Ala Gln Phe Gln
        210                 215                 220
Ala Glu Arg Ala Leu Tyr Glu Leu Pro Gly Leu Gln His Asp His Ile
225                 230                 235                 240
Val Arg Phe Ile Thr Ala Ser Arg Gly Gly Pro Gly Arg Leu Leu Ser
                245                 250                 255
Gly Pro Leu Leu Val Leu Glu Leu His Pro Lys Gly Ser Leu Cys His
            260                 265                 270
Tyr Leu Thr Gln Tyr Thr Ser Asp Trp Gly Ser Ser Leu Arg Met Ala
        275                 280                 285
Leu Ser Leu Ala Gln Gly Leu Ala Phe Leu His Glu Glu Arg Trp Gln
        290                 295                 300
Asn Gly Gln Tyr Lys Pro Gly Ile Ala His Arg Asp Leu Ser Ser Gln
305                 310                 315                 320
Asn Val Leu Ile Arg Glu Asp Gly Ser Cys Ala Ile Gly Asp Leu Gly
                325                 330                 335
Leu Ala Leu Val Leu Pro Gly Leu Thr Gln Pro Pro Ala Trp Thr Pro
            340                 345                 350
Thr Gln Pro Gln Gly Pro Ala Ala Ile Met Glu Ala Gly Thr Gln Arg
        355                 360                 365
Tyr Met Ala Pro Glu Leu Leu Asp Lys Thr Leu Asp Leu Gln Asp Trp
        370                 375                 380
Gly Met Ala Leu Arg Arg Ala Asp Ile Tyr Ser Leu Ala Leu Leu Leu
385                 390                 395                 400
Trp Glu Ile Leu Ser Arg Cys Pro Asp Leu Arg Pro Asp Ser Ser Pro
                405                 410                 415
Pro Pro Phe Gln Leu Ala Tyr Glu Ala Glu Leu Gly Asn Thr Pro Thr
            420                 425                 430
Ser Asp Glu Leu Trp Ala Leu Ala Val Gln Glu Arg Arg Arg Pro Tyr
```

```
                435                 440                 445
    Ile Pro Ser Thr Trp Arg Cys Phe Ala Thr Asp Pro Asp Gly Leu Arg
        450                 455                 460
    Glu Leu Leu Glu Asp Cys Trp Asp Ala Asp Pro Glu Ala Arg Leu Thr
    465                 470                 475                 480
    Ala Glu Cys Val Gln Gln Arg Leu Ala Ala Leu Ala His Pro Gln Glu
                485                 490                 495
    Ser His Pro Phe Pro Glu Ser Cys Pro Arg Gly Cys Pro Pro Leu Cys
                500                 505                 510
    Pro Glu Asp Cys Thr Ser Ile Pro Ala Pro Thr Ile Leu Pro Cys Arg
                515                 520                 525
    Pro Gln Arg Ser Ala Cys His Phe Ser Val Gln Gln Gly Pro Cys Ser
        530                 535                 540
    Arg Asn Pro Gln Pro Ala Cys Thr Leu Ser Pro Val
    545                 550                 555
```

```
<210> 12
<211> 17
<212> PRT
<213> Homo sapiens


<220>
<223> amino acid of the signal peptide of the human AMHR-II

<400> 12
Met Leu Gly Ser Leu Gly Leu Trp Ala Leu Leu Pro Thr Ala Val Glu
1                   5                   10                  15
Ala
```

**Description**

**FIELD OF THE INVENTION**

The present invention relates to a novel antibody directed against the human Anti-Müllerian Hormone type II receptor (AMHR-II) which is both Anti-Müllerian Hormone (AMH) competitive and antagonist, and to its uses thereof in therapeutic methods.

**BACKGROUND OF THE INVENTION**

Members of the transforming growth factor-beta (TGF-beta) superfamily are key regulators of various physiological processes. The Anti-Müllerian Hormone (AMH) is a 140 Kda glycoprotein, and a member of the TGF-beta superfamily, which is in particular well known for its role during male sexual differentiation (Josso N et al. Pediatr Endocrinol. Rev. 2006;3(4):347-358). In females, AMH is first secreted at a low level in the post-natal period by granulosa cells (GCs), then AMH levels surge during puberty before progressively declining throughout reproductive life until menopause (Visser et al. Reproduction 2006;131(1)1-9). Initiated in primary follicles, the AMH expression is strongest in pre-antral and small antral follicles and decreases subsequently up to ovulation. AMH is no longer detected in luteal bodies and atretic follicles.

To exert its effects, AMH binds to a specific type II receptor termed AMHR-II, which is co-expressed with AMH in GCs of growing follicles (Baarends et al. Endocrinology. 1995;136(11):4951-4962). During folliculogenesis, AMH inhibits the initial recruitment of primordial follicles and reduces the sensitivity of growing follicles to FSH. AMH indeed essentially functions as a gatekeeper for the rate of depletion of primordial follicles and selection of maturing follicles, utilizing a BMP-like signaling pathway throught AMHR-II and type I receptors, activating Smad 1/5/8. In addition, AMH is now recognized as an important clinical marker of the size of the ovarian follicle pool and as a predictor of the ovarian response to controlled ovarian hyperstimulation (Dewailly D et al. Hum Reprod Update. 2014;20(3)370-385). AMH concentration in follicular fluid is inversely correlated with granulosa cell proliferation, although normal physiology is disrupted with advancing age and in polycystic ovarian syndrome (PCOS).

The loop AMH/AMHR-II has been indeed described as playing a driving role in the physiopathology of PCOS (Pigny P et al., J Clin Endocrinol Metab 2003; 88(12): 5957-5962, Catteau-Jonard S et al., J Clin Endocrinol Metab 2008; 93 (11): 4456-4461, Dumont A et al., Reprod Biol Endocrinol 2015; 13: 137-146) either by its direct regulatory action on folliculogenesis in ovary, but also via an indirect pathway in hypothalamus involving an activation of the GnRH neurons LH pulsatility and secretion. Increased LH pulsatility is an important feature in many cases of PCOS (Cimino I et al., Nat. Commun. 2016; 7: 10055-10066).

PCOS is the most common female reproductive disorder, affecting 10-18% of women of reproductive age worldwide (Dewailly D. Best Pract Res Clin Obstet Gynaecol. 2016;37:5-11). The syndrome is in particular underpinned by excessive ovarian and/or adrenal androgen secretion. In non-pregnant women with PCOS, serum levels of AMH are 2-3-fold higher than in women without polycystic ovaries (PCO) and the severity of the reproductive dysfunction is positively correlated with AMH levels (Dewailly D et al. Hum Reprod. 2011;26(11):3123-3129); Piouka et al. (Am J Physiol Endocrinol Metab. 2009;296(2):E238-E243), supporting the hypothesis according to which AMH is involved in the follicular arrest observed in women with PCOS potentially by reducing the Granulosa cells sensitivity to FSH (Pellat L et al., Fertil Steril 2011; 92(1): 240-245).

In other gynecological disorders, the dual expression of AMH/AMHR-II has been described such as in endometriosis (Signorile PG et al., J Exp Clin Cancer Res 2014; 33(1): 46-54) and in myomas and adenomyosis of the uterus (Kim SY, et al. Obstet Gynecol Sci 2018; 61(1): 127-134).

In the field of cancer, the implication of AMHRII signaling is more complex. As mentioned above, AMHRII belongs to the TGFb receptor superfamily.

TGFb induces in general anti-proliferative responses in normal and cancer cells. Indeed, TGFbR2 and SMAD4 inactivating mutations and deletions are seen in various tumors and thought to be one of the triggers of the multistep tumorigenesis at early stages (Vogelstein et al., Science 2013;339(6127):1545-1558; Levy et al., Molec Cell biol 2005; 25(18) 8108-8125).

On the other hand, TGFb1 is highly expressed in tumor cells compared to the normal surrounding ones. This expression is correlated with tumor progression through enhancement of migration, invasion and survival of tumor cells at later stages as well as transducing immunosuppressive signals in the microenvironment (Morikawa et al., Cold Spring Harb. Perspect. 2016;8(5) : a021873 ; Luo et al., Transl. Oncol. 2019 12(3): 475-484; Furler et al.; Cancers 2018; 10(6), E199).

AMHRII is for its part reported in several types of gynecological cancers such as serous, clear cells, endometrioid, mucinous epithelial cancers, dysgerminomas, endometrial cancer, leiomyosarcomas and endometrial sarcomas (Bakkum-Gamez JN et al., Gynecol. Oncol. 2008; 108(1): 141-148; Kim SM et al., Oncol Letter 2019; 17(1):532-538) and cervical cancer (Taximaimaita R et al., Medicine 2018; 97(22): e10793).

AMH and AMHR-II are described as being implicated in the inhibition of cell cycle and as inducing apoptosis in cancers. Accordingly, AMH is known in the field of cancers as being a factor which protects the organism against cancer (M. Gowkielewicz et al.; Int. J. Mol. Sci. 2019;20(6):1325). As such, most of the current research is oriented toward facilitating, increasing or improving the effect of AMH in cancers, and thus its interaction with its receptor, as this interaction is known as triggering the signaling pathways of interest.

Besides, AMH and/or AMHRII expression was recently reported in various solid tumors other than gynecological.

Indeed, AMH expression was described in many solid tumors, such as gliomas, lung cancers, colorectal cancers, head and neck cancers, stomach cancers, pancreatic cancers, renal cancers, urothelial cancers, prostate cancers, testis cancers, breast cancers and in melanomas (Human Protein Atlas).

AMHR-II was also demonstrated as being expressed at the surface of various human cancer cells, which include especially colon cancer, lung cancer, hepatocellular carcinoma, testis cancer, pancreatic cancer, kidney cancer, breast cancer, thyroid cancer, gastric cancer, adrenal cancer, bladder cancer and prostate cancer. (Barret et al., Proc Amer Soc Clin Oncol, 37, Abst 774, 2018).

The present inventors have identified that, contrary to what is currently known and expected, inhibiting the interactions of AMH with its receptor AMHR-II leads to a reduction of AMHR-II expressing cells' viability and opens new therapeutic ways against diseases arising from the interaction between AMHR-II and the Anti-Müllerian Hormone (AMH).

Moreover, this is the first time that antagonizing the AMHRII pathway (using an antibody among other options) is demonstrated as an interesting and advantageous strategy against diseased cells expressing AMHR-II, and in particular as an anti-PCOS; anti-endometriosis; anti-premature menopause; and as an anti-cancer, in particular anti-non-gynecological tumors, therapeutic strategy.

Accordingly, there is a need in the art for further tools for the therapy of the above-mentioned diseases. There is more particularly a need for new tools allowing to specifically target cells expressing AMHR-II, and in particular diseased cells expressing AMHR-II. There is in particular a need for new tools allowing to specifically prevent and/or stop the signaling pathway triggered by the interaction between AMH and AMHR-II. Even more particularly, there is a need for new tools allowing to specifically prevent and/or stop the interaction between AMH and AMHR-II. There is in particular a need for new tools for the therapy of AMH and/or AMHR-II expressing cancers, and in particular AMHR-II expressing cancers, more particular AMHR-II expressing non-gynecological cancers.

The present invention provides a new tool able to respond to these needs.

## SUMMARY OF THE INVENTION

A first object of the present invention relates to an antagonist antibody, or a fragment thereof, that specifically binds to the extracellular domain of AMHR-II, in particular a competitive antagonist antibody that specifically binds to the extracellular domain of AMHR-II.

The inventors have indeed identified and demonstrated that AMHR-II is a target of interest in various pathologies, in particular those mentioned above, such as PCOS and cancers. As mentioned above, the inventors have in particular identified that it is of high interest, in many pathologies, to prevent the triggering of the signaling pathways starting from the activation of AMHR-II.

In particular, the antagonist antibody, or a fragment thereof, and more particularly the competitive antagonist antibody, or a fragment thereof, comprises:

(a) a heavy chain wherein the variable domain comprises:

- a H-CDR1 having a sequence set forth as SEQ ID NO: 3;
- a H-CDR2 having a sequence set forth as SEQ ID NO: 4; and
- a H-CDR3 having a sequence set forth as SEQ ID NO: 5;
  and

(b) a light chain wherein the variable domain comprises at least a CDR selected from the group consisting of:

- a L-CDR1 having a sequence set forth as SEQ ID NO: 8,
- a L-CDR2 having a sequence set forth as SEQ ID NO: 9; and
- a L-CDR3 having a sequence set forth as SEQ ID NO: 10.

In an embodiment, an antibody, or fragment thereof, according to the invention is non-immunogenic, and is in particular unable to bind to a Fc receptor (FcKO antibody).

In another embodiment, the antibody according to the invention comprises a heavy chain variable region having at least 85% identity with the sequence set forth as SEQ ID NO: 2:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

According to this embodiment, the heavy chain variable region of the antibody according to the invention can have at least 90%, in particular at least 95% identity, with the sequence set forth as SEQ ID NO: 2:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

In another embodiment, the antibody according to the invention comprises a light chain variable region having at least 85% identity with the sequence set forth as SEQ ID NO: 7:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

According to this embodiment, the light chain variable region of the antibody according to the invention can have at least 90%, in particular at least 95% identity, with the sequence set forth as SEQ ID NO: 7:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

In a further embodiment, the antibody according to the invention comprises a heavy chain having at least 85% identity with the sequence set forth as SEQ ID NO: 1:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

In particular, the heavy chain of the antibody according to the invention can have at least 90%, in particular at least 95% identity, with the sequence set forth as SEQ ID NO: 1:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

In another embodiment, the antibody according to the invention comprises a light chain having at least 85% identity with the sequence set forth as SEQ ID NO: 6:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

In particular, the light chain of the antibody according to the invention can have at least 90%, in particular at least 95% identity, with the sequence set forth as SEQ ID NO: 6:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

More particularly, the antibody according to the invention can comprise a heavy chain having the sequence set forth as SEQ ID NO: 1 and a light chain having the sequence set forth as SEQ ID NO: 6.
As mentioned above, the antibody according to the invention can be an antibody comprising a heavy chain having the sequence set forth as SEQ ID NO: 1 and a light chain having the sequence set forth as SEQ ID NO: 6, and further amended in order to be non-immunogenic, and in particular unable to bind to a Fc receptor (FcKO antibody).

In particular, the antibody, or fragment thereof, of the invention can be an antibody which competes for binding to the extracellular domain of AMHR-II with the antibody, or fragment thereof, comprising:

(a) a heavy chain wherein the variable domain comprises:

- a H-CDR1 having a sequence set forth as SEQ ID NO: 3;
- a H-CDR2 having a sequence set forth as SEQ ID NO: 4; and
- a H-CDR3 having a sequence set forth as SEQ ID NO: 5; and

(b) a light chain wherein the variable domain comprises at least a CDR selected from the group consisting of:

- a L-CDR1 having a sequence set forth as SEQ ID NO: 8,
- a L-CDR2 having a sequence set forth as SEQ ID NO: 9; and
- a L-CDR3 having a sequence set forth as SEQ ID NO: 10;

and in particular with an antibody defined on the basis of the sequences mentioned above.

In a further embodiment, an antibody's fragment of an antibody according to the invention can be selected from the group consisting of Fv, Fab, F(ab')2, Fab', dsFv, scFv, sc(Fv)2 and diabodies.

Another object of the present invention relates to a nucleic acid sequence encoding an antibody, or fragment thereof, of the invention.

A further object of the invention is a vector comprising a nucleic acid sequence according to the invention.

Another object of the invention is a host cell comprising the nucleic acid sequence or the vector of the invention.

The present invention also relates to an immunoconjugate comprising an antibody, or fragment thereof, according to the invention linked to a therapeutic agent.

Another object of the invention is a pharmaceutical composition comprising, in a pharmaceutically acceptable medium, at least one antibody, or fragment thereof, of the invention or at least one immunoconjugate of the invention.

The invention further relates to an antibody, or fragment thereof, of the invention, a cell of the invention, an immunoconjugate of the invention or a composition of the invention for its use as a drug.

In particular, the invention relates to an antibody, or fragment thereof, of the invention, a cell of the invention, an immunoconjugate of the invention or a composition of the invention for its use in the prevention and/or treatment of a disease arising from the interaction between AMHR-II and the Anti-Müllerian Hormone (AMH).

In an embodiment, the disease arising from the interaction between AMHR-II and the Anti-Müllerian Hormone (AMH) is selected from the group consisting of:

- Polycystic ovary syndrome (PCOS);
- Endometriosis;
- premature menopause; and
- cancers associated with the expression of AMHR-II, in particular selected from gynecologic cancers; colon cancer; lung cancer; hepatocellular carcinoma; testis cancer; pancreatic cancer; kidney cancer; breast cancer; thyroid cancer; gastric cancer; adrenal cancer; bladder cancer and prostate cancer.

The said disease can in particular be Polycystic ovary syndrome (PCOS). The disease can in particular be a cancer associated with the expression of AMHR-II.

In a particular embodiment, the antibody, or fragment thereof, the cell, the immunoconjugate or the composition of the invention is used in combination with another treatment, in particular another anti-cancer treatment.

## DESCRIPTION OF THE FIGURES

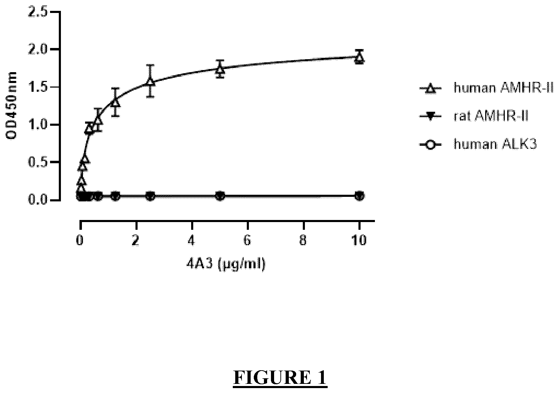

**Figure 1** illustrates the specific binding of anti-AMHR-II antibodies to the extracellular domain of human AMHR-II receptor, rat AMHR-II receptor and human ALK3 receptor.
Ordinate: Optical Density at 450nm; Abscissa: Quantity of antibody 4A3 ($\mu$g/mL).

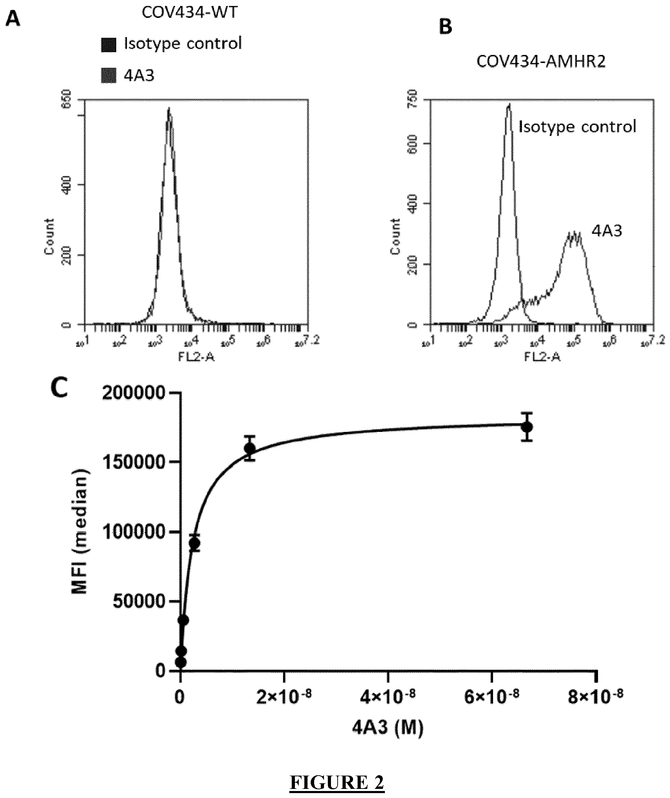

**Figure 2** illustrates the binding of the 4A3 antibody to the AMHR-II receptor expressed at the surface membrane of COV434-WT (A) and COV434-AMHR-II transfected cell line (B). Performed using a BD Accuri™ C6 Flow cytometer (BD Bioscience) (FL2-A setting).

Binding specificity was determined by FACS analysis at a concentration of 10μg/ml. (C) Binding curve of 4A3 antibody at different concentrations. Ordinate: Mean Fluorescence Intensity (MFI). Abscissa: Concentration of antibody 4A3 of the invention (M).

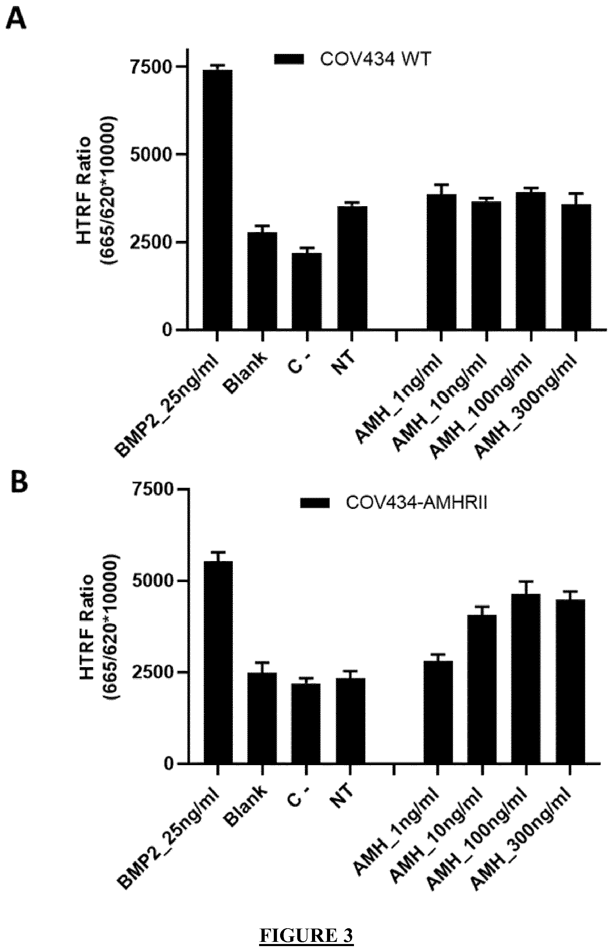

**Figure 3** validates the Smad pathway on COV434 cell lines. BMP2 induce Smad1 phosphorylation both in COV434-WT and COV434-AMHR-II transfected cell lines. A) No stimulation of Smad1 phosphorylation by AMH in COV434-WT cell line. B) Stimulation of Smad1 phosphorylation by AMH in COV434-AMHR-II transfected cell line. Ordinate: HTRF ratio (665/620*10000). Abscissa: from left to right: BMP2 at 25ng/mL; Blank (no treated cells without reagent); C- (negative control: no cells with reagents); NT (No Treatment); AMH used at 1ng/mL (AMH_1ng/mL); AMH used at 10ng/mL (AMH_10ng/mL); AMH used at 100ng/mL (AMH_100ng/mL); AMH used at 300ng/mL (AMH_300ng/mL).

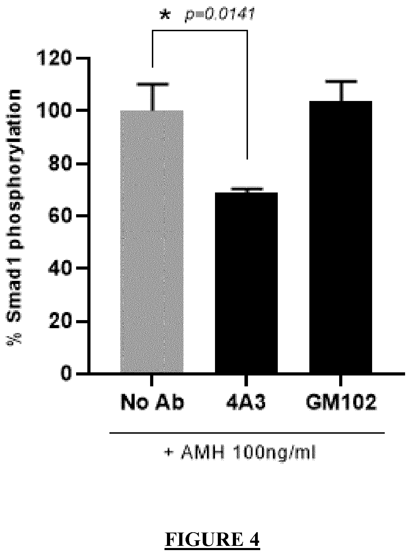

**Figure 4** illustrates antagonist/inhibitor effect of the antibodies 4A3 (according to the invention) and GM102 (outside of the invention) used at 100μg/ml on Smad1 phosphorylation induced by AMH at 100ng/ml. Ordinate: Smad1 phosphorylation percentage (%). Abscissa: from left to right: No Ab (No antibody) + AMH 100ng/mL; 4A3 (antibody of the invention) + AMH 100ng/mL; GM102 (antibody outside of the invention) + AMH 100ng/mL.

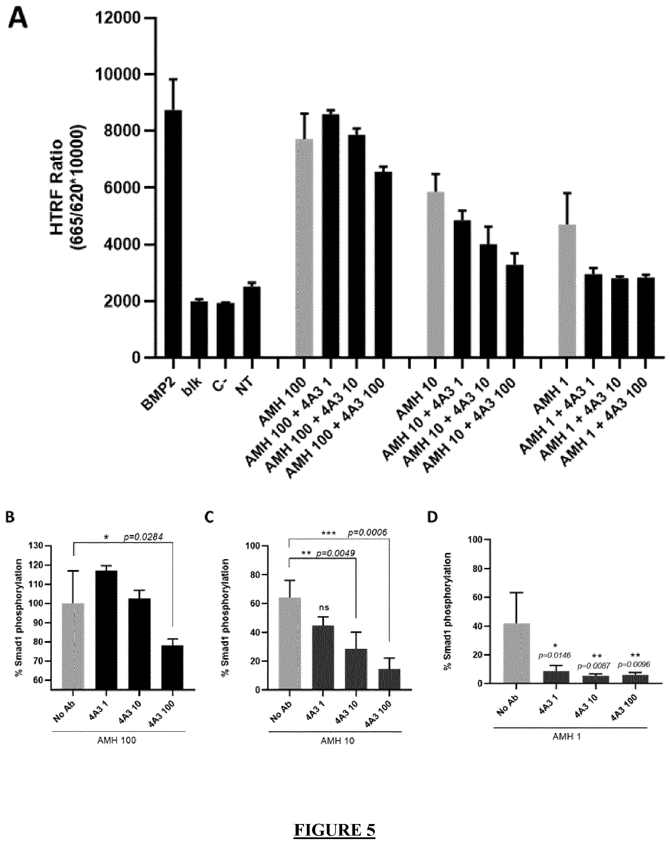

**Figure 5** illustrates antagonist/inhibitor effect of the antibody 4A3 on Smad1 phosphorylation induced by AMH. A) Ratio HTRF obtained with AMH at 1ng/ml, 10ng/ml and 100ng/ml and the competitive effect of antibody 4A3 at concentrations 1μg/ml, 10μg/ml and 100μg/ml at each AMH concentration. **B), C), D)** represent the percentage of Smad1 phosphorylation (%) depending the concentration of AMH used and the concentration of 4A3 antagonist antibody. AMH used at 100ng/mL - AMH 100 (B); 10ng/mL - AMH 10 (C) or 1ng/mL - AMH 1 (D). 4A3 used at 1ng/mL (4A3 1), 10ng/mL (4A3 10) or 100ng/mL (4A3 100). No Ab: negative control.

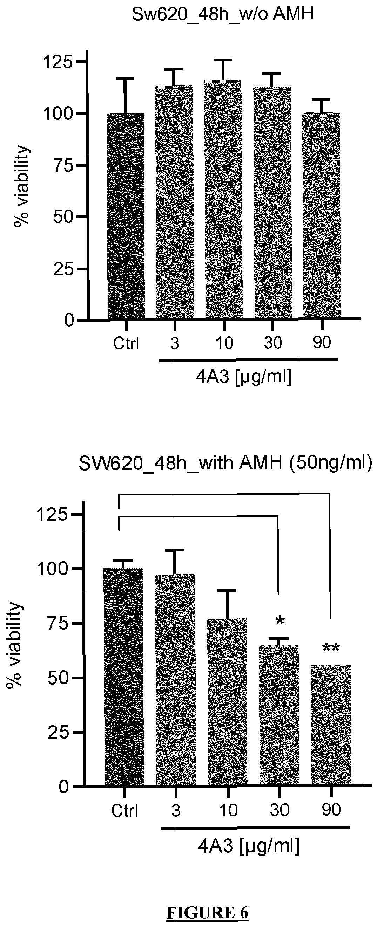

**Figure 6** illustrates the viability percentage (%) of SW620 cancer cells (Dukes' type C, colorectal adenocarcinoma), that naturally express AMHR-II on their surface, when exposed, for 48 hours, to difference concentrations of antibody 4A3 (1, 3, 10, 30 or 90 μg/mL) A) in the absence of AMH or B) in the presence of 50ng/mL of AMH. A negative control (Ctrl - no antibody) is also represented.

## DETAILED DESCRIPTION OF THE INVENTION

As mentioned above, the inventors have unexpectedly determined that contrary to what was known until now, it is of high interest in many diseases to target AMHR-II in order to prevent, in the diseased cells, the signaling pathways triggered by the activation of AMHR-II.

The inventors have moreover identified an antagonist anti-AMHR-II antibody which is competitive to AMH. This antibody is in particular specific for the human AMHR-II.

Accordingly, in the present text, the term "AMHR-II" denotes the Anti-Müllerian Hormone type II Receptor, and in particular the human Anti-Müllerian Hormone type II Receptor. The AMH receptor (AMHR or AMHR-II) is a serine/threonine kinase with a single transmembrane domain belonging to the family of type II receptors for TGF-beta-related proteins. Type II receptors bind the ligand on their own but require the presence of a type I receptor for signal transduction. Imbeaud et al. (1995, Nature Genet, Vol. 11: 382-388,) cloned the human AMH type II receptor gene. The sequence of the human AMHR-II is described as SEQ ID NO: 11 herein (lacking the signal peptide MLGSLGLWALLPTAVEA (SEQ ID NO: 12).

SEQ ID NO: 11 corresponds to the following amino acid sequence:

PPNRRTCVFFEAPGVRGSTKTLGELLDTGTELPRAIRCLYSRCCFGIWNLTQDRAQVE

MQGCRDSDEPGCESLHCDPSPRAHPSPGSTLFTCSCGTDFCNANYSHLPPPGSPGTPG

SQGPQAAPGESIWMALVLLGLFLLLLLLLLGSIILALLQRKNYRVRGEPVPEPRPDSGR

DWSVELQELPELCFSQVIREGGHAVVWAGQLQGKLVAIKAFPPRSVAQFQAERALYE

LPGLQHDHIVRFITASRGGPGRLLSGPLLVLELHPKGSLCHYLTQYTSDWGSSLRMAL

SLAQGLAFLHEERWQNGQYKPGIAHRDLSSQNVLIREDGSCAIGDLGLALVLPGLTQ

PPAWTPTQPQGPAAIMEAGTQRYMAPELLDKTLDLQDWGMALRRADIYSLALLLWE

ILSRCPDLRPDSSPPPFQLAYEAELGNTPTSDELWALAVQERRRPYIPSTWRCFATDPD

GLRELLEDCWDADPEARLTAECVQQRLAALAHPQESHPFPESCPRGCPPLCPEDCTSI

PAPTILPCRPQRSACHFSVQQGPCSRNPQPACTLSPV

The terms "anti-AMHR-II antibody" refers to an antibody directed against AMHR-II, in particular against the extracellular domain of AMHR-II. In particular, the terms "anti-human-AMHR-II antibody" refers to an antibody directed against human AMHR-II, in particular against the extracellular domain of human AMHR-II.

According to the present invention, "antibody" or "immunoglobulin" have the same meaning and will be used equally in the present text. The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also variants (including derivatives) of antibodies. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CHI, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and
H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

Accordingly, in the present text, the term "antibody" is used in the broadest sense and includes fully assembled antibodies, monoclonal antibodies, polyclonal antibodies, multispecific antibodies (e.g., bispecific antibodies), antibody fragments that can bind AMHR-II, and recombinant peptides comprising the forgoing as long as they exhibit the desired biological activity.

Antibody fragments according to the invention can in particular be selected from the group consisting of Fv, Fab, F(ab')2, Fab', dsFv, scFv, sc(Fv)2 and diabodies. They may be produced by recombinant DNA techniques or by enzymatic or chemical cleavage of intact antibodies, according to methods well known to the man skilled in the art.

Due to their small size, these antibody fragments can be of high interest for example through their binding to the AMHR-II receptor at the hypothalamic level on the GnRH neurons, a major component of PCOS physiopathology.

"Single-chain Fv" or "scFv" antibody fragments comprise the VH and VL domains of antibody, wherein these domains are present in a single polypeptide chain. Generally, the Fv polypeptide further comprises a polypeptide linker between the VH and VL domains, which enables the scFv to form the desired structure for antigen binding. For a review of scFv, see Pluckthun in The Pharmacology of Monoclonal Antibodies, Vol 113, Rosenburg and Moore eds. Springer-Verlag, New York, pp. 269-315 (1994).

The term "diabodies" refers to small antibody fragments with two antigen-binding sites, which fragments comprise a heavy chain variable domain (VH) connected to a light chain variable domain (VL) in the same polypeptide chain (VH and VL). By using a linker that is too short to allow pairing between the two domains on the same chain, the domains are forced to pair with the complementary domains of another chain and create two antigen- binding sites. Diabodies are described more fully in, for example, EP 404,097; WO 93/11161; and Hollinger et al., Proc. Natl. Acad. Sci. USA, 90:6444-6448 (1993).

Diabodies or bi-specific antibodies can be roughly divided into two categories: immunoglobulin G (IgG)-like molecules and non-IgG-like molecules. IgG-like bsAbs retain Fc-mediated effector functions such as antibody-dependent cell-mediated cytotoxicity (ADCC), complement-dependent cytotoxicity (CDC), and antibody-dependent cellular phagocytosis (ADCP) (Spiess et al., 2015, Mol Immunol., Vol. 67(2) : 95-106.). The Fc region of bsAbs facilitates purification and improves solubility and stability. Bi-specific antobodies in IgG-like formats usually have longer serum half-lives owing to their larger size and FcRn-mediated recycling (Kontermann et al., 2015, Bispecific antibodies. Drug Discov Today Vol. 20(7) : 838-47). Non-IgG-like bsAbs are smaller in size, leading to enhanced tissue penetration (Kontermann et al., 2015, Bispecific antibodies. Drug Discov Today Vol. 20(7): 838-47).

A monoclonal antibody according to the invention can in particular be a chimeric, humanized or human antibody.

The monoclonal antibodies specified herein specifically include "chimeric" anti-AMHR-II antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81 :6851-6855 (1984)).

The monoclonal antibodies specified herein also encompass humanized anti-AMHR-II antibodies. "Humanized" forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). Humanized antibodies may be produced by obtaining nucleic acid sequences encoding CDR domains and constructing a humanized antibody according to techniques known in the art. Methods for producing humanized antibodies based on conventional recombinant DNA and gene transfection techniques are well known in the art (See, e.g., Riechmann L. *et al* 1988; Neuberger M S. *et al*. 1985). Antibodies can be humanized using a variety of techniques known in the art including, for example, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan E A (1991); Studnicka G M et *al*. (1994); Roguska M A. et *al*. (1994)), and chain shuffling (U.S. Pat. No. 5,565,332). The general recombinant DNA technology for preparation of such antibodies is also known (see European Patent Application EP 125023 and International Patent Application WO 96/02576).

The monoclonal anti-AMHR-II antibodies specified herein further encompass anti-AMHR-II human antibodies. A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. Proc. Natl. Acad. Sci. 95:6157-6162 (1998)); Hoogenboom and Winter, J. Mol. Biol, 227:381 (1991); Marks et al., J. Mol. Biol, 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., J. Immunol, 147 (1):86-95 (1991); and U.S. Pat. No. 5,750,373.

The terms "monoclonal antibody" as used herein refer to an antibody obtained from a population of substantially homogeneous antibodies, i.e., the individual antibodies comprising the population are identical except for possible naturally occurring mutations or alternative post-translational modifications that may be present in minor amounts. Monoclonal antibodies are highly specific; in contrast to conventional (polyclonal) antibody preparations that typically include different antibodies directed against different determinants (epitopes), each monoclonal antibody is directed against a single determinant on the antigen. In addition to their specificity, the monoclonal antibodies are advantageous in that they are synthesized by the homogeneous culture, uncontaminated by other immunoglobulins with different specificities and characteristics. For example, monoclonal antibodies in accordance with the invention may be made by the hybridoma method first described by Kohler et al., Nature 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature 352:624-628 (1991) or Marks et al., J. Mol. Biol. 222:581-597 (1991), for example.

As used herein, "antibody mutant" or "antibody variant" refers to an amino acid sequence variant of the species-dependent antibody wherein one or more of the amino acid residues of the species-dependent antibody have been modified. Such mutants necessarily have less than 100% sequence identity or similarity with the species-dependent antibody but maintain CDRs with 100% sequence identity with the CDRs of an antibody according to the invention, in the present case with an antibody comprising:

(a) a heavy chain wherein the variable domain comprises:

- a H-CDR1 having a sequence set forth as SEQ ID NO: 3;
- a H-CDR2 having a sequence set forth as SEQ ID NO: 4; and
- a H-CDR3 having a sequence set forth as SEQ ID NO: 5;
and

(b) a light chain wherein the variable domain comprises at least a CDR selected from the group consisting of:

- a L-CDR1 having a sequence set forth as SEQ ID NO: 8;
- a L-CDR2 having a sequence set forth as SEQ ID NO: 9; and
- a L-CDR3 having a sequence set forth as SEQ ID NO: 10.

These sequences are indicated here-after:

SEQ ID NO: 3 (H-CDR1): GFTFSNNAMN

SEQ ID NO: 4 (H-CDR2): YISGSSRYIS

SEQ ID NO: 5 (H-CDR3): SSDDYFGGGMDV

SEQ ID NO: 8 (L-CDR1): AGTSSDVGGDNDVS

SEQ ID NO: 9 (L-CDR2): YDSYRPS

SEQ ID NO: 10 (L-CDR3): SSSTYYSTRV

Identity or similarity with respect to a sequence as mentioned in the present text is defined herein as the percentage of amino acid residues in the candidate sequence that are identical (i.e. same residue) or similar (i.e. amino acid residue from the same group based on common side-chain properties, see below) with the species-dependent antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

A competitive antibody according to the present invention means an antibody which competes for binding AMHR-II on the same epitope as the Anti-Müllerian Hormone (AMH).

An "antagonist antibody" is meant an antibody molecule that is able to inhibit the activation of AMHR-II, and in particular able to inhibit the triggering of the signal pathway(s) activated by the interaction of AMH to AMHR-II.

An "antibody heavy chain," as used herein, refers to the larger of the two types of polypeptide chains present in all antibody molecules in their naturally occurring conformations.

An "antibody light chain," as used herein, refers to the smaller of the two types of polypeptide chains present in all antibody molecules in their naturally occurring conformations, κ and λ light chains refer to the two major antibody light chain isotypes.

As used herein the term "complementarity determining region" or "CDR" refers to the part of the two variable chains of antibodies (heavy and light chains) that recognize and bind to the particular antigen. The CDRs are the most variable portion of the variable chains and provide the antibody with its specificity. There are three CDRs on each of the variable heavy (VH) and variable light (VL) chains and thus there are a total of six CDRs per antibody molecule. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a VHCDR3 is located in the variable domain of the heavy chain of the antibody in which it is found, whereas a VLCDR1 is the CDR1 from the variable domain of the light chain of the antibody in which it is found. An antibody that binds LHR will have a specific VH region and the VL region sequence, and thus specific CDR sequences. Antibodies with different specificities (i.e. different combining sites for different antigens) have different CDRs. Although it is the CDRs that vary from antibody to antibody, only a limited number of amino acid positions within the CDRs are directly involved in antigen binding. These positions within the CDRs are called specificity determining residues (SDRs).

"Framework regions" (hereinafter FR) are those variable domain residues other than the CDR residues. Each variable domain typically has four FRs identified as FR1, FR2, FR3 and FR4.

The term "treatment" or "therapy" refers to administering an antibody, a cell or a composition according to the invention with the purpose to cure, heal, alleviate, relieve, alter, remedy, ameliorate, improve, or affect a disease according to the invention, the symptoms of the condition, or to prevent or delay the onset of the symptoms, complications, biochemical indicia of a disease, or otherwise arrest or inhibit further development of the disease, condition, or disorder in a statistically significant manner.

According to the invention, the term "patient" or "patient in need thereof' is intended for a human or non-human mammal affected or likely to be affected with a disease as defined in the present text, in particular with a disease arising from the interaction between AMHR-II and the Anti-Müllerian Hormone (AMH).

In the present text, "preventing" means reducing the risk of manifestation of a phenomenon.

An antibody according to the invention is in particular used in a therapeutically effective amount. By a "*therapeutically effective amount*" of the antibody of the invention is meant a sufficient amount of the antibody to treat the disease considered, at a reasonable benefit/risk ratio applicable to any medical treatment. The same applies mutated mutandis to a composition and/or to a cell according to the invention. It will be understood, however, that the total daily usage of the antibodies, cells and compositions of the present invention will be decided by the attending physician within the scope of sound medical judgment. The specific therapeutically effective dose level for any particular patient will depend upon a variety of factors including the disorder being treated and the severity of the disorder; the activity of the specific antibody employed; the specific composition employed; the specific cell employed; the age, body weight, general health, sex and diet of the patient; the time of administration, route of administration, and rate of excretion of the specific antibody employed; the duration of the treatment; the drugs used in combination or coincidental with the specific antibody employed; and like factors well known in the medical arts. For example, it is well known within the skill of the art to start doses of the compound at levels lower than those required to achieve the desired therapeutic effect and to gradually increase the dosage until the desired effect is achieved.

As used herein, the percent identity between two amino acid sequences can be determined using the algorithm of E. Meyers and W. Miller (Comput. Appl. Biosci., 4:1 1-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453, 1970) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1, 2, 3, 4, 5, or 6.

By "non-immunogenic" according to the invention, it means that an antibody according to the invention, in a particular embodiment, is not capable of eliciting a humoral or cellular immune response, and preferably both, in an animal having a functional immune system. Accordingly, an antibody according to the invention can be unable to bind a Fc receptor. Such an antibody can be termed "Fcγ ablation variants" or "Fc knock out" (FcKO or KO) variants. Indeed, such variant is amended in order to remove the normal binding of the Fc domain to one or more or all of the Fcγ receptors (e.g. FcγR1, FcγRIIa, FcγRIIb, FcγRIIIa, etc.). Methods to obtain such antibody variants are well known in the art, such as for example illustrated in Yamane-Ohnuki et al. (Biotechnol Bioeng 2004; 87(5): 614-622) and Satoh et al. (Expert Opin Biol Ther 2006; 6(11): 1161-1173).

Instead, in an advantageous embodiment, solely the ability of the antibody of the invention to bind to AMHR-II, and even more to compete with AMH to bind to AMHR-II, and to antagonize the cell signaling pathway activated by the binding of AMH to AMHR-II is required.

As used herein, "gynecologic" cancers are in particular selected in the group consisting of ovarian cancer, cervical cancer, endometrial cancer, gestational trophoblastic disease cancer (choriocarcinoma), uterine sarcoma, vaginal cancer, vulvar cancer and Fallopian tube cancer.

AMHR-II is expressed at the cell membrane of gynecologic and non-gynecologic cancer tissues, as well as at the cell membrane of tissues of different non-cancer diseases as mentioned in the present text, with a variable frequency depending of the cancer/disease type which is considered. Illustratively, AMHR-II is expressed more frequently by cancer cells derived from tumor tissue originating from patients affected with colorectal cancer than by cancer cells derived from tumor tissue originating from patients affected with a head and neck cancer. This means that these two types of cancers are eligible for an anti-cancer treatment targeting AMHR-II, but that such an anti-cancer treatment will be less frequently relevant for treating patients affected with a head and neck cancer.

Any disease mentioned in the present text may be treated by an AMHR-II-binding antibody according to the invention, or a fragment thereof, provided that disease cells from the said disease express AMHR-II at their membrane, thus provided that the presence of AMHR-II proteins at the disease cell membrane can be detected or determined according to any method well known in the art.

Thus, an antibody according to the invention, or a fragment thereof, as well as cells and/or compositions according to the invention, is effective for treating a plurality of distinct kinds of diseases, and in particular of different types of tumors, provided that the AMHR-II target protein is expressed at the disease cells membrane.

Incidentally, in particular in the field of anti-cancer active ingredients consisting of target-binding molecules, e.g. target-binding antibodies, the situation wherein the same active ingredient is effective for treating a plurality of distinct cancers is not unprecedented. Illustratively, the anti-PDI antibody named pembrolizumab has been authorized by the US Food and Drug Administration (FDA) as an active ingredient useful in the treatment of a variety of distinct kinds of cancers, provided that the said cancers share the same physiological features. The same applies in view of diseases according to the invention different from a cancer.

Thus, an individual affected with a disease may be treated for the said disease with an AMHR-II-binding agent as described herein when AMHR-II membrane expression by the disease cells previously collected from the said individual is detected or otherwise determined by an appropriate method.

In some embodiments, expression of AMHR-II at the cell membrane of disease cells encompasses that the said disease cells express AMHR-II at a given quantifiable level or higher than the said quantifiable level.

Thus, according to some embodiments, responsiveness of an individual affected with a disease according to the invention to a treatment with an antibody of the invention, or a fragment thereof, may be assessed by determining whether disease cells from a sample previously collected from the said individual express AMHR-II at their membrane.

According to some embodiments, responsiveness of an individual affected with a disease according to the invention to a treatment with an antibody according to the invention, or a fragment thereof, or a cell or a composition of the invention, may be assessed by determining whether disease cells from a sample previously collected from the said individual express AMHR-II at their membrane above a determined threshold value.

The AMHR-II membrane expression level that may be used in some embodiments for determining the responsiveness of a patient affected with a disease according to the invention to a treatment with an antibody of the invention, or a fragment thereof, or a cell or a composition of the invention, may be assessed with a variety of techniques, which include (i) the percentage of disease cells contained in a disease sample that express AMHR-II at their membrane, (ii) the mean number of AMHR-II proteins at the disease cell membrane and (iii) the FACS AMHR-II signal profile of the disease cells contained in a tested disease cell sample.

According to some embodiments, disease cells comprised in a tumor sample previously collected for an individual affected with a disease according to the invention may be assessed as expressing membranous AMHR-II when membranous AMHR-II is detected in 5% or more of the disease cells comprised in the said disease sample.

Thus, in some embodiments, an individual affected with a disease according to the invention is determined as being responsive to a treatment with an antibody of the invention, or a cell or a composition of the invention, when 5% or more of the disease cells comprised in a disease sample previously collected from the said individual express AMHR-II at their membrane.

Methods for determining the frequency (e.g. the percentage) of disease cells, and in particular of cancer cells, expressing membrane AMHR-II proteins are well known in the art (see for example WO2018189379).

According to some embodiments, responsiveness of a patient affected with a disease according to the invention to a disease treatment with an antibody of the invention, or a cell or a composition of the invention may be assessed by determining the mean number of AMHR-II proteins present at the membrane of the disease cells contained in a disease sample previously collected from the said patient.

In some embodiments, a patient affected with a disease according to the invention may be classified as responsive to a treatment with an antibody of the invention, or a cell or a composition of the invention when the mean number of membrane AMHR-II proteins expressed by the disease cells contained in a disease sample previously collected from the said patient is of 10 000 AMHR-II proteins or more.

Assessing the number of AMHR-II proteins expressed at the disease cell membrane may be performed by using conventional methods comprising (a) a step of incubating a sample containing the cells from a disease tissue sample previously collected from the patient with a detectable compound that binds specifically with AMHR-II protein, such as a fluorescently labeled anti-AMHR-II antibody, and further (b) a step of determining the number of the said detectable compounds, e.g. the number of fluorescently labeled anti-AMHR-II antibodies, bound to each tested cell from the said sample. Assessing the number of AMHR-II proteins expressed at the disease cell membrane may be, for instance, performed by using the well-known Fluorescence Activated Cell Sorting (FACS) technique, as it is shown in the examples herein.

In still other embodiments, a patient affected with a disease may be classified as responsive to a treatment with an antibody of the invention, or a cell or a composition of the invention by analysis of the AMHR-II FACS profile of the disease cells contained in a disease sample previously collected from the said patient.

According to these still other embodiments, a patient affected with a disease according to the invention may be classified as responsive to a treatment with an antibody of the invention, or a cell or a composition of the invention when, in a method of fluorescence activated cell sorting (FACS), the ratio of (i) the mean fluorescence intensity of the disease cells incubated with an anti-AMHR-II fluorescently labeled antibody to (ii) the mean fluorescence intensity (MFI) value obtained from disease cells incubated with an isotypic fluorescently labeled antibody is of 1.5 or more.

For determining the said mean fluorescence intensity ratio, both the isotypic antibody and the anti-AMHR-II antibody are labeled with the same fluorescent agent, such as the Alexa Fluor 488 dye commercialized by the Company ThermoFisher Scientific., as shown in WO2018189379.

In some further embodiments, responsiveness of a disease individual to a treatment with an antibody of the invention, or a fragment thereof, or a cell or a composition of the invention may be determined by calculating an AMHR-II expression score allowing to discriminate between (i) membrane AMHR-II-expressing disease cells derived from diseases that may be treated with an AMHR-II-binding agent and (ii) membrane AMHR-II-expressing disease cells derived from diseases that may not be treated with an AMHR-II-binding agent.

Thus, the inventors have determined that patients affected with a disease of the present text, who are especially eligible to a disease treatment with an antibody of the invention, or a fragment thereof, or a cell or a composition of the invention, encompass those having diseases expressing AMHR-II at the cell membrane at a sufficiently high level for consisting in relevant cell targets to be destroyed.

Then, according to these further embodiments, the inventors have determined that a minimal AMHR-II expression level measured in a disease cell sample from a disease patient may confirm that the said patient is responsive to a treatment with an antibody of the invention, or a fragment thereof, or a cell or a composition of the invention, and that the said patient may thus be treated by an antibody, a cell or a composition described here-in.

Responsiveness of an individual affected with a disease to a treatment with an antibody of the invention, or a fragment thereof, or a cell or a composition of the invention, may thus also be determined when AMHR-II expression level by disease cells comprised in a sample previously collected from the said individual is assessed by both determining (i) the frequency of disease cells expressing membranous AMHR-II, e.g. the percentage of tumor cells expressing AMHR-II at their membrane and (ii) the level of AMHR-II membrane expression by the said disease cells, e.g. the mean number of membranous AMHR-II proteins per cell.

Thus, in some of these further embodiments, responsiveness of a patient affected with a disease according to the invention to an antibody of the invention, or a fragment thereof, or a cell or a composition of the invention, in a sample of disease cells previously collected from the said patient, may be assessed by determining that (i) the disease cells contained in the said sample exhibit a minimal mean number of human AMHR-II proteins at their membrane and that (ii) the frequency of the cells expressing human AMHR-II at their membrane, e.g. the percentage of cells expressing human AMHR-II at their membrane, is of at least a threshold value.

Accordingly, it is also described herein a further method that may also be used for determining a specific AMHR-II expression score value allowing to discriminate between (i) disease patients that are not eligible to a disease treatment with an antibody of the invention, or a cell or a composition of the invention, an antibody of the invention, or a fragment thereof, or a cell or a composition of the invention, i.e. disease patients that are not responsive to a disease treatment with an antibody of the invention, or a fragment thereof, or a cell or a composition of the invention, and (ii) disease patients that are eligible to a disease treatment with an antibody of the invention, or a fragment thereof, or a cell or a composition of the invention, i.e. disease patients that are responsive to a disease treatment with an antibody of the invention, or a fragment thereof, or a cell or a composition of the invention.

According to the above embodiments of determining responsiveness of an individual affected with a disease according to the invention to a treatment with an antibody of the invention, or a fragment thereof, or a cell or a composition of the invention, a membranous AMHR-II expression score can be determined, for a given disease of the invention cell sample, by taking into account both (i) the frequency of AMHR-II-expressing cells in the said disease cell sample and (ii) the level of AMHR-II expression by the said AMHR-II-expressing cells. Typically, an AMHR-II expression score of a given disease cell sample can be determined by the following formula (I) :

$$E\text{-}SCORE = FREQ \times AMHR\text{-}II\_LEVEL,$$

wherein

- E-SCORE means the AMHR-II expression score value for a given disease cell sample,

- FREQ means the frequency of the cells contained in the said disease cell sample for which membrane AMHR-II expression is detected, and

- AMHR-II_LEVEL means the level of expression of AMHR-II by the AMHR-II-expressing cells contained in the said given disease cell sample.

Illustratively, a E-SCORE of 1.0 is determined for a given disease according to the invention cell sample wherein (i) 50% of the cells express AMHR-II (FREQ value of 0.5) and (ii) the AMHR-II expression level (AMHR-II_LEVEL) is of 2.

In particular embodiments, an AMHR-II expression score (or E-SCORE) is determined by immunohistological methods. According to these embodiments, AMHR-II membrane expression is assessed by using a detectable antibody specific for AMHR-II and by (i) determining the frequency of cells having the said anti-AMHR-II antibody bound thereto and (ii) determining the intensity of the signal generated by the said detectable anti-AMHR-II antibody after its binding to the membrane-expressed AMHR-II. In such a method, the said antibody specific for AMHR-II can for example be an antibody according to the invention.

Thus, according to embodiments of the above method, patients affected with a disease described herein and who may be treated against disease with an antibody of the invention, or a cell or a composition of the invention, may be preferably those for which an AMHR-II expression score is of 1.0 or more has been determined, which includes those for which an AMHR-II expression score is of 1.5 or more has been determined.

Although, AMHR-II-expressing disease cells having an AMHR-II expression score of 1.5 or more have been determined for various diseases, and in particular for various cancers, albeit to distinct frequencies. Illustratively, the inventors have shown herein that cancer cells derived from colon tumors are classified as AMHR-II positive (i.e. having an AMHR-II score of 1.5 or more) with a higher frequency than cancer cells derived from head and neck cancer.

For determining the level of AMHR-II membrane expression, detection of AMHR-II at the cell membrane shall be most preferably performed by using an anti-AMHR-II monoclonal antibody having a high affinity and high specificity for AMHR-II.

Further, determination of AMHR-II expression by an immuno-histochemical method with the view of determining a AMHR-II score most preferably involves a careful pretreatment of the tissue sample before contacting the said sample with an appropriate detection reagent (e.g. a high affinity anti-AMHR-II monoclonal antibody such as monoclonal 3C23K antibody, having a Kd value of 55.3pM for binding to AMHR-II). Sample pretreatment shall allow increasing the availability to the detection reagent of the AMHR-II molecules expressed at the cell surface. Illustratively, staining method comprises an appropriate combination of specific steps such as (i) a high-temperature dewaxing by exposure to a microwave source and (ii) a system for amplifying the signal generated by the binding of an AMHR-II-binding reagent, such as a biotinylated anti-AMHR-II antibody that may be subsequently complexed with a streptavidin-conjugated detectable reagent. A pretreatment dewaxing step has appeared to be important for reversing the detection signal extinction effect due to the prior tissue fixation step. The inventors have shown that AMHR-II detectability is particularly sensitive to the action of formalin which is used for the tissue fixation step.

In the context of the present invention, this means that an antibody of the invention, or a fragment thereof, or a cell or a composition of the invention, will be a useful therapeutic agent with a higher frequency for treating patients affected with a colon cancer than for treating patients affected with a head and neck cancer. This also means that, although an antibody of the invention, or a cell or a composition of the invention, may be a relevant therapeutic agent for treating patients affected with head and neck cancer, it will be preferred to test previously for the AMHR-II expression of the tumor-derived cancer cells for deciding that a specific patient will be administered with an antibody of the invention, or a fragment thereof, or a cell or a composition of the invention.

Thus, the inventors have shown herein that the antibody of the invention targeting AMHR-II, but also having an antagonist effect as well as being competitive to AMH for binding to AMHR-II are useful as novel therapeutic tools for preventing or treating a disease arising from the interaction between AMHR-II and the Anti-Müllerian Hormone (AMH), and in particular a disease as mentioned in the present text.

*AMHR-II binding antibody of the invention*

As mentioned previously, a first object of the invention is an antagonist antibody, or a fragment thereof, that specifically binds to the extracellular domain of AMHR-II.

In particular, the said antagonist antibody, or a fragment thereof, is a competitive antagonist antibody.

More particularly, a competitive antagonist antibody of the invention comprises:

(a) a heavy chain wherein the variable domain comprises:

- a H-CDR1 having a sequence set forth as SEQ ID NO: 3;
- a H-CDR2 having a sequence set forth as SEQ ID NO: 4; and
- a H-CDR3 having a sequence set forth as SEQ ID NO: 5;
  and

(b) a light chain wherein the variable domain comprises at least a CDR selected from the group consisting of:

- a L-CDR1 having a sequence set forth as SEQ ID NO: 8;
- a L-CDR2 having a sequence set forth as SEQ ID NO: 9; and
- a L-CDR3 having a sequence set forth as SEQ ID NO: 10.

A heavy chain variable region of an antibody according to the invention in particular has at least 85% identity with the sequence set forth as SEQ ID NO: 2:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

SEQ ID NO: 2 corresponds to the following amino acid sequence:

EVQLVESGGSLVKPGGSLRLSCAASGFTFSNNAMNWVRQAPGKGLEWISYISGSSRYI
SYADFVKGRFTISRDNAKNSLYLQMNSLRAEDTAVYYCVRSSDDYFGGGMDVWGR
GTLVTVSS

More particularly, the heavy chain variable region of an antibody according to the invention can have at least 90% identity with the sequence set forth as SEQ ID NO: 2:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

In a particular embodiment, the heavy chain variable region of an antibody according to the invention can have at least 90%, at least 91%, at least 92%, at least 93%, at least 94%, at least 95%, at least 96%, at least 97%, at least 98% or at least 99% identity, with the sequence set forth as SEQ ID NO: 2:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

In a more particular embodiment, the heavy chain variable region of an antibody according to the invention has the sequence set forth as SEQ ID NO: 2.

In a particular embodiment, the heavy chain of an antibody according to the invention has at least 85% identity with the sequence set forth as SEQ ID NO: 1:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

SEQ ID NO: 1 corresponds to the following amino acid sequence:

EVQLVESGGSLVKPGGSLRLSCAASGFTFSNNAMNWVRQAPGKGLEWISYISGSSRYI
SYADFVKGRFTISRDNAKNSLYLQMNSLRAEDTAVYYCVRSSDDYFGGGMDVWGR
GTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKRVEPKSCDKTHT
CPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK

More particularly, the heavy chain of an antibody according to the invention has at least 90% identity with the sequence set forth as SEQ ID NO: 1:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

More particularly, the heavy chain of an antibody according to the invention has at least 90%, at least 91%, at least 92%, at least 93%, at least 94%, at least 95%, at least 96%, at least 97%, at least 98% or at least 99% identity, with the sequence set forth as SEQ ID NO: 1:

- the H-CDR1 having 100% identity with the sequence set forth as SEQ ID NO: 3;
- the H-CDR2 having 100% identity with the sequence set forth as SEQ ID NO: 4; and
- the H-CDR3 having 100% identity with the sequence set forth as SEQ ID NO: 5.

In a more particular embodiment, the heavy chain of an antibody according to the invention has the sequence set forth as SEQ ID NO: 1.
A light chain variable region of an antibody according to the invention in particular has at least 85% identity with the sequence set forth as SEQ ID NO: 7:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

SEQ ID NO: 7 corresponds to the following amino acid sequence:

QSVLTQPASVSGSPGQSITISCAGTSSDVGGDNDVSWYQQHPGKAPKLMIYYDSYRPS
GVSNRFSGSKSGNTASLTISGLQAEDEADYYCSSSTYYSTRVFGGGTKLTVL

More particularly, the light chain variable region of an antibody according to the invention can have at least 90% identity with the sequence set forth as SEQ ID NO: 7:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

In a particular embodiment, the light chain variable region of an antibody according to the invention can have at least 90%, at least 91%, at least 92%, at least 93%, at least 94%, at least 95%, at least 96%, at least 97%, at least 98% or at least 99% identity, with the sequence set forth as SEQ ID NO: 7:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

In a more particular embodiment, the light chain variable region of an antibody according to the invention has the sequence set forth as SEQ ID NO: 7.

In a particular embodiment, the light chain of an antibody according to the invention has at least 85% identity with the sequence set forth as SEQ ID NO: 6:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

SEQ ID NO: 6 corresponds to the following amino acid sequence:

QSVLTQPASVSGSPGQSITISCAGTSSDVGGDNDVSWYQQHPGKAPKLMIYYDSYRPS

GVSNRFSGSKSGNTASLTISGLQAEDEADYYCSSSTYYSTRVFGGGTKLTVLGQPKA

APSVTLFPPSSEELQANKATLVCLISDFYPGAVTVAWKADSSPVKAGVETTTPSKQSN

NKYAASSYLSLTPEQWKSHRSYSCQVTHEGSTVEKTVAPTECS

More particularly, the light chain of an antibody according to the invention has at least 90% identity with the sequence set forth as SEQ ID NO: 6:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

More particularly, the light chain of an antibody according to the invention has at least 90%, at least 91%, at least 92%, at least 93%, at least 94%, at least 95%, at least 96%, at least 97%, at least 98% or at least 99% identity with the sequence set forth as SEQ ID NO: 6:

- the L-CDR1 having 100% identity with the sequence set forth as SEQ ID NO: 8;
- the L-CDR2 having 100% identity with the sequence set forth as SEQ ID NO: 9; and
- the L-CDR3 having 100% identity with the sequence set forth as SEQ ID NO: 10.

In a more particular embodiment, the light chain of an antibody according to the invention has the sequence set forth as SEQ ID NO: 6.

In an embodiment of the invention, the heavy chain variable region of an antibody according to the invention has the sequence set forth as SEQ ID NO: 2 and the light chain variable region of the antibody has the sequence set forth as SEQ ID NO: 7.

In particular, an antibody according to the invention can be such that it comprises a heavy chain having the sequence set forth as SEQ ID NO: 1 and a light chain having the sequence set forth as SEQ ID NO: 6.

In an embodiment, an antibody, or fragment thereof, according to the invention is non-immunogenic. In particular, an antibody, or fragment thereof, according to the invention can be unable to bind to a Fc receptor.

As previously mentioned, an antibody fragment according to the invention can in particular be selected from the group consisting of Fv, Fab, F(ab')2, Fab', dsFv, scFv, sc(Fv)2 and diabodies.

An antibody according to the invention can be produced by any technique known in the art, such as, without limitation, any chemical, biological, genetic or enzymatic technique, either alone or in combination.

Knowing the amino acid sequence of the desired sequence, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, antibodies of the invention can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

An antibody according to the invention, or its fragments thereof, can moreover be an antibody, or fragment thereof, which competes for binding to the extracellular domain of AMHR-II with the antibody defined as comprising:

(a) a heavy chain wherein the variable domain comprises:

- a H-CDR1 having a sequence set forth as SEQ ID NO: 3;
- a H-CDR2 having a sequence set forth as SEQ ID NO: 4; and
- a H-CDR3 having a sequence set forth as SEQ ID NO: 5;
  and

(b) a light chain wherein the variable domain comprises at least a CDR selected from the group consisting of:

- a L-CDR1 having a sequence set forth as SEQ ID NO: 8;
- a L-CDR2 having a sequence set forth as SEQ ID NO: 9; and
- a L-CDR3 having a sequence set forth as SEQ ID NO: 10;

in particular with the antibody whose heavy chain variable region has the sequence set forth as SEQ ID NO: 2 and light chain variable region has the sequence set forth as SEQ ID NO: 7;
and more particularly with the antibody whose heavy chain has the sequence set forth as SEQ ID NO: 1 and light chain has the sequence set forth as SEQ ID NO: 6.

Nucleic acid sequence

Accordingly, a further object of the invention relates to a nucleic acid sequence encoding an antibody according to the invention, or a fragment thereof. The terms "nucleic acid" and "nucleic acid sequence" are used interchangeably in the present text.

Typically, said nucleic acid is a DNA or RNA molecule, which may be included in any suitable vector, such as a plasmid, cosmid, episome, artificial chromosome, phage or a viral vector.

Vectors

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

So, a further object of the invention relates to a vector comprising a nucleic acid of the invention.

Such vectors may comprise regulatory elements, such as a promoter, enhancer, terminator and the like, to cause or direct expression of said antibody upon administration to a subject. Examples of promoters and enhancers used in the expression vector for animal cell include early promoter and enhancer of SV40 (Mizukami T. *et al.* 1987), LTR promoter and enhancer of Moloney mouse leukemia virus (Kuwana Y *et al.* 1987), promoter (Mason JO *et al.* 1985) and enhancer (Gillies SD *et al.* 1983) of immunoglobulin H chain and the like.

Any expression vector for animal cell can be used, so long as a gene encoding the human antibody C region can be inserted and expressed. Examples of suitable vectors include pAGE107 (Miyaji H *et al.* 1990), pAGE103 (Mizukami T *et al.* 1987), pHSG274 (Brady G *et al.* 1984), pKCR (O'Hare K *et al.* 1981), pSG1 beta d2-4-(Miyaji H *et al.* 1990) and the like.

Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

Host cells

The present invention also relates to a host cell which has been transfected, infected or transformed by a nucleic acid and/or a vector according to the invention. Accordingly, a further object of the invention relates to a host cell comprising a nucleic acid sequence of the invention or a vector of the invention.

The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence, and in particular the antibody, or fragment thereof, of the invention. A host cell that receives and expresses introduced DNA or RNA bas been "transformed".

The nucleic acids of the invention may be used to produce an antibody of the invention, or a fragment thereof, in a suitable expression system. The term "expression system" means a host cell and compatible vector under suitable conditions, e.g. for the expression of a protein coded for by foreign DNA carried by the vector and introduced to the host cell.

Common expression systems include E. coli host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G *et al.*; 1980), rat YB2/3HL.P2.G11.16Ag.20 cell (ATCC CRL1662, hereinafter referred to as "YB2/0 cell"), and the like.

Immunoconjugates

Another object of the invention relates to an immunoconjugate comprising an antibody, or a fragment thereof, of the invention linked to a therapeutic agent.

Antibodies according to the present invention can indeed be conjugated to a cytotoxic agent such as a chemotherapeutic agent, toxin (e.g. an enzymatically active toxin of bacterial, fungal, plant or animal origin, or fragments thereof), or a radioactive isotope (i.e., a radio conjugate). Such antibody conjugates encompass those described in the PCT application n° WO 2017/025458.

Cytotoxic agents encompass enzymatically active toxins. Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha- sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes.

A variety of radionuclides are available for the production of radioconjugate antibodies.

Conjugates of the antibody and cytotoxic agent can be made using a variety of bifunctional protein coupling agents such as those disclosed in the PCT application n° WO 2017/025458.

Immunoconjugates according to the invention can in particular be an antibody-drug conjugate (ADC).

Antibody-drug conjugates (ADC)

In another aspect, the present invention provides an anti-AMHR-II antibody-drug conjugate, in particular an anti-human-AMHR-II antibody-drug-conjugate, and more particularly an anti-human-AMHR-II monoclonal antibody drug-conjugate.

An anti-AMHR-II antibody-drug conjugate according to the invention is intended to be used in the treatment of cancers, and in particular in the treatment of the cancers mentioned in the present text.

An "anti-AMHR-II antibody-drug conjugate" as used herein refers to an anti-AMHR-II antibody, or a fragment thereof, according to the invention conjugated to a therapeutic agent.

Such anti-human-AMHR-II antibody-drug conjugates (ADC) produce clinically beneficial effects on AMHR-II-expressing cells when administered to a subject in particular a subject with an AMHR-II-expressing cancer, typically when administered alone but also in combination with other therapeutic agents.

In typical embodiments, an antibody of the invention can be conjugated to a cytotoxic agent, such that the resulting antibody-drug conjugate (ADC) exerts a cytotoxic or cytostatic effect on a AMHR-II-expressing cancer cells when taken up or internalized by the cell. Particularly suitable moieties (therapeutic agents) for conjugation to antibodies are chemotherapeutic agents, prodrug converting enzymes, radioactive isotopes or compounds, or toxins. For example, an anti-AMHR-II antibody, or fragment thereof, can be conjugated to a cytotoxic agent such as a chemotherapeutic agent or a toxin (e.g., a cytostatic or cytocidal agent such as, for example, abrin, ricin A, pseudomonas exotoxin, or diphtheria toxin).

Useful classes of cytotoxic agents include, for example, antitubulin agents, auristatins, DNA minor groove binders, DNA replication inhibitors, alkylating agents (e.g., platinum complexes such as cis-platin, mono(platinum), bis(platinum) and tri-nuclear platinum complexes and-carboplatin), anthracyclines, antibiotics, antifolates, antimetabolites, chemotherapy sensitizers, duocarmycins, etoposides, fluorinated pyrimidines, ionophores, lexitropsins, nitrosoureas, platinols, pre-forming compounds, purine antimetabolites, puromycins, radiation sensitizers, steroids, taxanes, topoisomerase inhibitors, vinca alkaloids, or the like.

Individual cytotoxic agents include, for example, an androgen, anthramycin (AMC), asparaginase, 5-azacytidine, azathioprine, bleomycin, busulfan, buthionine sulfoximine, camptothecin, carboplatin, carmustine (BSNU), CC-1065 (Li et al., Cancer Res. 42:999-1004, 1982), chlorambucil, cisplatin, colchicine, cyclophosphamide, cytarabine, cytidine arabinoside, cytochalasin B, dacarbazine, dactinomycin (formerly actinomycin), daunorubicin, decarbazine, docetaxel, doxorubicin, an estrogen, 5-fluordeoxyuridine, etopside phosphate (VP-16), 5-fluorouracil, gramicidin D, hydroxyurea, idarubicin, ifosfamide, irinotecan, lomustine (CCNU), mechlorethamine, melphalan, 6-mercaptopurine, methotrexate, mithramycin, mitomycin C, mitoxantrone, nitroimidazole, paclitaxel, plicamycin, procarbizine, streptozotocin, tenoposide (VM-26), 6-thioguanine, thioTEPA, topotecan, vinblastine, vincristine, and vinorelbine.

Particularly suitable cytotoxic agents include, for example, dolastatins (e.g., auristatin E, AFP, MMAF, MMAE), DNA minor groove binders (e.g., enediynes and lexitropsins), duocarmycins, taxanes (e.g., paclitaxel and docetaxel), puromycins, vinca alkaloids, CC-1065, SN-38 (7-ethyl-10-hydroxy-camptothein), topotecan, morpholino-doxorubicin, rhizoxin, cyanomorpholino-doxorubicin, echinomycin, combretastatin, netropsin, epothilone A and B, estramustine, cryptophysins, cemadotin, maytansinoids, discodermolide, eleutherobin, and mitoxantrone.

In certain embodiments, a cytotoxic agent is a conventional chemotherapeutic such as, for example, doxorubicin, paclitaxel, melphalan, vinca alkaloids, methotrexate, mitomycin C or etoposide. In addition, potent agents such as CC-1065 analogues, calicheamicin, maytansine, analogues of dolastatin 10, rhizoxin, and palytoxin can be linked to an anti-HER3-expressing antibody.

In specific variations, the cytotoxic or cytostatic agent is auristatin E (also known in the art as dolastatin-10) or a derivative thereof. Typically, the auristatin E derivative is, e.g., an ester formed between auristatin E and a keto acid. For example, auristatin E can be reacted with paraacetyl benzoic acid or benzoylvaleric acid to produce AEB and AEVB, respectively. Other typical auristatin derivatives include AFP (dimethylvaline-valine-dolaisoleuine-dolaproine-phenylalanine-p-phenylenediamine), MMAF (dovaline-valine-dolaisoleunine-dolaproine-phenylalanine), and MAE (monomethyl auristatin E). The synthesis and structure of auristatin E and its derivatives are described in U.S. Patent Application Publication No. 20030083263; International Patent Publication Nos. WO 2002/088172 and WO 2004/010957; and U.S. Patent Nos. 6,884,869; 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414.

In other variations, the cytotoxic agent is a DNA minor groove binding agent. (See, e.g., U.S. Patent No. 6,130,237.) For example, in certain embodiments, the minor groove binding agent is a CBI compound. In other embodiments, the minor groove binding agent is an enediyne (e.g., calicheamicin).

In certain embodiments, an antibody-drug conjugate (ADC) comprises an anti-tubulin agent. Examples of anti-tubulin agents include, for example, taxanes (e.g., Taxol® (paclitaxel), Taxotere® (docetaxel)), T67 (Tularik), vinca alkyloids (e.g., vincristine, vinblastine, vindesine, and vinorelbine), and dolastatins (e.g., auristatin E, AFP, MMAF, MMAE, AEB, AEVB). Other antitubulin agents include, for example, baccatin derivatives, taxane analogs (e.g., epothilone A and B), nocodazole, colchicine and colcimid, estramustine, cryptophysins, cemadotin, maytansinoids, combretastatins, discodermolide, and eleutherobin. In some embodiments, the cytotoxic agent is a maytansinoid, another group of anti-tubulin agents. For example, in specific embodiments, the maytansinoid is maytansine or DM-1 (ImmunoGen, Inc.; see also Chari et al., Cancer Res. 52:127-131, 1992).

In other embodiments, the cytotoxic agent is an antimetabolite. The antimetabolite can be, for example, a purine antagonist (e.g., azothioprine or mycophenolate mofetil), a dihydrofolate reductase inhibitor (e.g., methotrexate), acyclovir, gangcyclovir, zidovudine, vidarabine, ribavarin, azidothymidine, cytidine arabinoside, amantadine, dideoxyuridine, iododeoxyuridine, poscarnet, or trifluridine.

In other embodiments, an anti-AMHR-II antibody, or fragment thereof, is conjugated to a prodrug converting enzyme. The pro-drug converting enzyme can be recombinantly fused to the antibody or chemically conjugated thereto using known methods. Exemplary pro-drug converting enzymes are carboxypeptidase G2, β-glucuronidase, penicillin-V-amidase, penicillin-G-amidase, β-lactamase, β-glucosidase, nitroreductase and carboxypeptidase A.

Techniques for conjugating therapeutic agents to proteins, and in particular to antibodies or fragment thereof, are well-known. (See, e.g., Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Deiker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. See also, e.g., PCT publication WO 89/12624.).

Linkers

Typically, the antibody-drug conjugate compounds comprise a linker unit between the drug unit and the antibody unit (i.e. antibody of fragment thereof). In some embodiments, the linker is cleavable under intracellular conditions, such that cleavage of the linker releases the drug unit from the antibody, or a fragment thereof, in the intracellular environment. In yet other embodiments, the linker unit is not cleavable and the drug is released, for example, by antibody degradation.

In some embodiments, the linker is cleavable by a cleaving agent that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside target cells (see, e.g., Dubowchik and Walker, 1999, Pharm. Therapeutics 83:67-123).

Most typical are peptidyl linkers that are cleavable by enzymes that are present in 191P4D12-expressing cells. Examples of such linkers are described, e.g., in U.S. Pat. No. 6,214,345, incorporated herein by reference in its entirety and for all purposes. In a specific embodiment, the peptidyl linker cleavable by an intracellular protease is a Val-Cit linker or a Phe-Lys linker (see, e.g., U.S. Pat. No. 6,214,345, which describes the synthesis of doxorubicin with the Val-Cit linker). One advantage of using intracellular proteolytic release of the therapeutic agent is that the agent is typically attenuated when conjugated and the serum stabilities of the conjugates are typically high.

In other embodiments, the cleavable linker is pH-sensitive, i.e., sensitive to hydrolysis at certain pH values.

Typically, the pH-sensitive linker hydrolyzable under acidic conditions. For example, an acid-labile linker that is hydrolyzable in the lysosome (e.g., a hydrazone, semicarbazone, thiosemicarbazone, cis-aconitic amide, orthoester, acetal, ketal, or the like) can be used. (See, e.g., U.S. Pat. Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, e.g., a thioether attached to the therapeutic agent via an acylhydrazone bond (see, e.g., U.S. Pat. No. 5,622,929).

In yet other embodiments, the linker is cleavable under reducing conditions (e.g., a disulfide linker). A variety of disulfide linkers are known in the art, including, for example, those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene), SPDB and SMPT. (See, e.g., Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987. See also U.S. Pat. No. 4,880,935.)

In yet other specific embodiments, the linker is a malonate linker (Johnson et al., 1995, Anticancer Res. 15:1387-93), a maleimidobenzoyl linker (Lau et al., 1995, Bioorg-Med-Chem. 3(10):1299-1304), or a 3'-N-amide analog (Lau et al., 1995, Bioorg-Med-Chem. 3(10):1305-12).

In yet other embodiments, the linker unit is not cleavable and the drug is released by antibody degradation.

Typically, the linker is not substantially sensitive to the extracellular environment. As used herein, "not substantially sensitive to the extracellular environment," in the context of a linker, means that no more than about 20%, typically no more than about 15%, more typically no more than about 10%, and even more typically no more than about 5%, no more than about 3%, or no more than about 1% of the linkers, in a sample of antibody-drug conjugate compound, are cleaved when the antibody-drug conjugate compound is present in an extracellular environment (e.g., in plasma). Whether a linker is not substantially sensitive to the extracellular environment can be determined, for example, by incubating with plasma the antibody-drug conjugate compound for a predetermined time period (e.g., 2, 4, 8, 16, or 24 hours) and then quantitating the amount of free drug present in the plasma.

Pharmaceutical composition

Another object of the present invention is a pharmaceutical composition comprising, in a pharmaceutically acceptable medium, at least one antibody, or fragment thereof, of the invention or at least one immunoconjugate of the invention.

Indeed, for its administration, an antibody, or fragment thereof, of the invention or an immunoconjugate of the invention is preferably formulated as a pharmaceutical composition.

A pharmaceutical composition of the invention can be formulated according to known methods to prepare pharmaceutically useful compositions, whereby the therapeutic molecule is combined in a mixture with a pharmaceutically acceptable medium.

A "*pharmaceutically acceptable medium*" refers to a non-toxic material that is compatible with a biological system such as a cell, a cell culture, a tissue or an organism. In particular, a pharmaceutical composition is said to comprise a "pharmaceutically acceptable medium" if its administration can be tolerated by a recipient patient. Sterile phosphate-buffered saline is one example of a pharmaceutically acceptable carrier. Other suitable carriers are well-known to those in the art. (See, e.g., Gennaro (ed.), Remington's Pharmaceutical Sciences (Mack Publishing Company, 19th ed. 1995).) Formulations may further include one or more excipients, preservatives, solubilizers, buffering agents, albumin to prevent protein loss on vial surfaces, etc.

The form of the pharmaceutical composition of the invention, the route of administration, the dosage and the regimen naturally depend upon the condition to be treated, the severity of the illness, the age, weight, and sex of the patient, etc.

The pharmaceutical compositions of the invention can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the antibody or immunoconjugate may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

Carriers

An antibody, a fragment thereof, or immunoconjugate of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The antibodies or immunoconjugate of the invention may be formulated within a therapeutic mixture to comprise about 0.0001 to 1.0 milligrams, or about 0.001 to 0.1 milligrams, or about 0.1 to 1.0 or even about 50 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration; time release capsules; and any other form currently used.

In certain embodiments, the use of liposomes and/or nanoparticles is contemplated for the introduction of antibodies into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 $\mu$m) are generally designed using polymers able to be degraded in vivo. Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 $\mu$m. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

Therapeutic applications

Another object of the invention relates to an antibody, or fragment thereof, of the invention, a cell of the invention or a composition of the invention for its use as a drug.

In particular, an object of the invention relates to an antibody, or fragment thereof, of the invention, a cell of the invention or a composition of the invention for its use in the prevention and/or treatment of a disease arising from the interaction between AMHR-II and the Anti-Müllerian Hormone (AMH).

As already disclosed elsewhere in the present specification, the antibodies, fragments thereof, immunoconjugates, cells and compositions of the invention may be used for preventing or treating any disease arising from the interaction between AMHR-II and AMH, since as demonstrated in the present text:

- the signaling pathway(s) triggering by the interaction between AMHR-II and AMH have a pro-disease effect in the pathologies considered in the present text, and in particular have a pro-oncogenic effect in cancers expressing AMHR-II on their surface; and

- an antibody, or a fragment thereof, according to the invention (i) specifically recognizes AMHR-II, (ii) has competitive properties in view of AMH and (iii) has antagonist properties as defined above.

In particular, the said disease arising from the interaction between AMHR-II and AMH can be a Polycystic ovary syndrome (PCOS) (Pierre A et al., J Clin Endocrin Metab 2017; 102(11): 3970-3978).

The said disease can also be endometriosis (Signorile et al., J Exp Clin Cancer 2014; 33:46:1-9) or premature menopause (Victoria et al., J Gynecol Hum Reprod 2019; 48:19-24).

The said disease can moreover be selected from cancers associated with the expression of AMHR-II (i.e. cancers whose cancer cells express on their surface AMHR-II).

Such cancers can be gynecologic cancers or non-gynecologic cancers.

The said cancers associated with the expression of AMHR-II can in particular be selected from gynecologic cancers; colon cancer; lung cancer; hepatocellular carcinoma; testis cancer; pancreatic cancer; kidney cancer; breast cancer; thyroid cancer; gastric cancer; adrenal cancer; bladder cancer and prostate cancer. (Barret et al., Proc AACR Annual Meeting, 2018, Abst 774).

In a particular embodiment, the cancers associated with the expression of AMHR-II mentioned above are non-gynecologic cancers, in particular selected from the group consisting of colon cancer; lung cancer; hepatocellular carcinoma; testis cancer; pancreatic cancer; kidney cancer; breast cancer; thyroid cancer; gastric cancer; adrenal cancer; bladder cancer and prostate cancer.

In some embodiments, patients according to the invention are tested for determining whether their disease cells, and in particular whether their cancer cells, express AMHR-II at their surface, before performing a treatment with an antibody, a fragment thereof, immunoconjugate, cell or composition according to the invention.

The disease cells that are used generally originate from a biopsy tissue sample that has previously been collected from the said patient.

In some embodiments, the antibody, a fragment thereof, immunoconjugate, cell or composition according to the invention is used as the sole active ingredient against the targeted disease, such as for example as the sole anti-cancer active ingredient.

In some other embodiments, the treatment of the disease with the said antibody, a fragment thereof, immunoconjugate, cell or composition according to the invention also comprises subjecting the said individual to one or more further treatment(s) against the disease of interest, such as for example one or more further anti-cancer treatment(s) or anti-cancer agent(s).

An "anticancer treatment" can for example comprise a radiotherapy treatment.

An "anticancer agent" is defined as any molecule that can either interfere with the biosynthesis of macromolecules (DNA, RNA, proteins, etc.) or inhibit cellular proliferation, or lead to cell death by apoptosis or cytotoxicity for example. Among the anticancer agents, there may be mentioned alkylating agents, topoisomerase inhibitors and intercalating agents, anti-metabolites, cleaving agents, agents interfering with tubulin, monoclonal antibodies.

Other anti-cancer agents that may be used in combination with an antibody, a fragment thereof, immunoconjugate, cell or composition according to the invention encompass paclitaxel or a platinum salt such as oxaliplatin, cisplatin and carboplatin.

The anti-cancer agent may also be selected from chemotherapeutic agents other than the platinum salts, small molecules, monoclonal antibodies or else anti-angiogenesis peptibodies, these agents being different from the objects of the present invention as detailed above.

The chemotherapeutic agents other than the platinum salts include the intercalating agents (blocking of DNA replication and transcription), such as the anthracyclines (doxorubicin, pegylated liposomal doxorubicin), the topoisomerase inhibitors (camptothecin and derivatives: Karenitecin, topotecan, irinotecan), or else SJG-136, the inhibitors of histone deacetylase (vorinostat, belinostat, valproic acid), the alkylating agents (bendamustine, glufosfamide, temozolomide), the anti-mitotic plant alkaloids, such as the taxanes (docetaxel, paclitaxel), the vinca alkaloids (vinorelbine), the epothilones (ZK-Epothilone, ixabepilone), the anti-metabolites (gemcitabine, elacytarabine, capecitabine), the kinesin spindle protein (KSP) inhibitors (ispinesib), trabectedin or else ombrabulin (combretastatin A-4 derivative).

Among the small molecules there are the poly(ADP-ribose)polymerase (PARP) inhibitors: olaparib, iniparib, veliparib, rucaparib, CEP-9722, MK-4827, BMN-673, the kinase inhibitors, such as the tyrosine kinase inhibitors (TKI) among which there may be mentioned the anti-VEGFR molecules (sorafenib, sunitinib, cediranib, vandetanib, pazopanib, BIBF 1120, semaxanib, Cabozantinib, motesanib), the anti-HER2/EGFR molecules (erlotinib, gefitinib, lapatinib), the anti-PDGFR molecules (imatinib, BIBF 1120), the anti-FGFR molecules (BIBF 1120), the aurora kinase/tyrosine kinase inhibitors (ENMD-2076), the Src/Abl kinase inhibitor (Saracatinib), or also Perifosine, Temsirolimus (mTOR inhibitor), alvocidib (cyclin-dependent kinase inhibitor), Volasertib (inhibitor of PLK1 (polo-like kinase 1) protein, LY2606368 (inhibitor of checkpoint kinase 1 (chk 1), GDC-0449 (Hedgehog Pathway Inhibitor), Zibotentan (antagonist of the ETA-receptor), Bortezomib, Carfilzomib (proteasome inhibitor), cytokines such as IL-12, IL-18, IL-21, INF-alpha, INF-gamma.

Among the antibodies, there may be mentioned, the anti-VEGF: bevacizumab, the anti-VEGFR: ramucirumab, the anti-HER2/EGFRs: trastuzumab, pertuzumab, cetuximab, panitumumab, MGAH22, matuzumab, anti-PDGFR alpha: IMC-3G3, the anti-folate receptor: farletuzumab, the anti-CD27: CDX-1127, the anti-CD56: BB-10901, the anti-CD105: TRC105, the anti-CD276: MGA271, the anti-AGS-8: AGS-8M4, the anti-DR5: TRA-8, the anti-HB-EGF: KHK2866, the anti-mesothelins: amatuximab, BAY 94-9343 (immunotoxin), catumaxomab (EpCAM/CD3 bispecific antibody), the anti-IL2R: daclizumab, the anti-IGF-1R: ganitumab, the anti-CTLA-4: ipilimumab, the anti-PDI: nivolumab and pembrolizumab, the anti-CD47: Weissman B6H12 and Hu5F9, Novimmune 5A3M3, INHIBRX 2A1, Frazier VxP037-01LC1 antibodies, the anti-Lewis Y: Hu3S193, SGN-15 (immunotoxin), the anti-CA125: oregovomab, the anti-HGF: rilotumumab, the anti-IL6: siltuximab, the anti-TR2: tigatuzumab, the anti-alpha5 beta1 integrin: volociximab, the anti-HB-EGF: KHK2866. The anti-angiogenesis peptibodies are selected from AMG 386 and CVX-241.

The antibody, immunoconjugate, cell or composition according to the invention and the additional treatment against the targeted disease may be combined within one and the same pharmaceutical composition, or may be used in the form of separate pharmaceutical compositions, which may be administered simultaneously or sequentially. In particular, the products may be administered separately, namely either concomitantly, or independently, for example with a time gap.

The invention also concerns the use of an antibody, a fragment thereof, immunoconjugate, cell or composition according to the invention for the preparation of a medicament for preventing or treating a disease arising from the interaction between AMHR-II and the Anti-Müllerian Hormone (AMH) as defined above.

This invention also pertains to a method for preventing or treating a disease arising from the interaction between AMHR-II and the Anti-Müllerian Hormone (AMH) as defined above, wherein the said method comprises a step of administering to an individual in need thereof an antibody, a fragment thereof, immunoconjugate, cell or composition according to the invention.

Kits

Finally, the invention also provides kits comprising at least one antibody, fragment thereof, immunoconjugate, cell or composition of the invention. Kits containing at least one of these objects find can for example find use in detecting AMHR-II expression, or in therapeutic or diagnostic assays. Kits of the invention can contain an antibody coupled to a solid support, e.g., a tissue culture plate or beads (e.g., sepharose beads). Kits can be provided which contain antibodies for detection and quantification of AMHR-II *in vitro,* e.g. in an ELISA or a Western blot. Such antibody useful for detection may be provided with a label such as a fluorescent or radiolabel.

The present invention is further illustrated by, without in any way being limited to, the examples below.

## EXAMPLES

### Example 1: BINDING OF ANTI-AMHR-II ANTIBODIES TO THE EXTRACELLULAR DOMAIN OF AMHR-II RECEPTOR

The binding capacity of the anti-AMHR-II 4A3 antibody was evaluated by ELISA. ELISA plates (96 wells, Maxisorp, Nunc) were coated overnight at +4°C with 100ng per well of human AMHR-II-ECD-Fc, rat AMHR-II-ECD-Fc or human ALK3-ECD-Fc (R&D Systems).

Coated plates were blocked with PBS-BSA 2% for 1 hour at 37°C and washed three times with PBS-Tween20 0.05%. The anti-AMHR-II 4A3 antibody was serially diluted in PBST-BSA1% and incubated for 1 hour at 37°C. The bound anti-AMHR-II 4A3 was detected by adding HRP-conjugated F(ab')2 goat anti-human F(ab')2 fragment specific (Interchim) diluted at 1:10000 and incubated for one hour at 37°C. Revelation was done with TMB (Sigma) and the reaction was stopped by adding $H_2SO_4$ 1N (v:v). Absorbances were measured at 450nm and binding data curves were analysed using GraphPad Prism 8 software.

Figure 1 shows an example of the binding curves of the 4A3 antibody.

No cross-reactivity was detected for rat AMHR-II-ECD-Fc and human ALK3-ECD-Fc (negative control). EC50 of 4A3 antibody for human AMHR-II was 6nM (+/-2).

### Example 2: BINDING OF ANTI-AMHR-II ANTIBODIES TO THE AMHR-II RECEPTOR AT THE SURFACE OF TRANSFECTED CELL LINE

The COV434 AMHR-II transfected and the COV434 WT cell lines were maintained in DMEM/GlutaMax (Gibco) supplemented with 10% FBS, penicillin 100U/ml and Streptomycin 100μg/ml. Geneticin at 666μg/ml was added for the COV434 AMHR-II tranected cell line.

For Fluorescent-activated cell sorting (FACS) analysis, $3 \times 10^5$ cells were incubated for 1 hour at 4°C with 10μg/ml of 4A3 antibody (figure 2A) or with a range of 4A3 concentrations (from 67nM to 0.2pM, figure 2B). After washes, Phycoerythrin-conjugated anti-human IgG antibody (1:200, Interchim) was added for 1 hour at 4°C. FACS analysis of the resuspended cells was done on a BD Accuri™ C6 flow cytometer (BD Bioscience). Binding affinity were determined using GraphPad Prism 8 with "one-site specific binding" fit equation.

Figures 2A shows the specific binding of 4A3 antibody on COV434 AMHR-II transfected cell line. No unspecific binding was detected on COV434 WT.

Figures 2B shows an example of the binding curve for a range of 4A3 concentrations. The Kd apparent was 2.1 ± 0.5 nM.

## Example 3: ANTIBODY 4A3 MEDIATE INHIBITION OF SMAD1 PHOSPHORYLATION INDUCED BY AMH IN COV434-AMHR-II CELL LINE

The level of Smad1 phosphorylation was assessed using Homogeneous Time Resolved Fluorescence (HTRF) method cell assay. The phospho-Smadl Ser463/465 kit provided by CisBio (Catalog number 63ADK063PEG) was used to quantity the level of phosphorylation of Smadlin COV434 cell lines.

Briefly, 25.000 cells were plated in triplicate in a 96-well plate. After the cells adhered to the plate, medium was replaced to DMEM medium without red phenol supplemented with 0.2% of serum (SVF). After overnight incubation the assay procedure was done in serum-free medium without red phenol. Cells were then incubated in a total volume of 100 $\mu$L/well of medium, containing or not the different component AMH (Cter AMH, R&D Systems), BMP2 (R&D Systems) or mixture of AMH/antibody 4A3 at different concentrations. Cells were incubated at 37°C during 1 hour and then lysed in 50 $\mu$L/well of the supplemented lysis buffer for 45 min at room temperature with shaking. A volume of 16 $\mu$L of each lysate was transferred into a 96-well low volume white plate and made up to the final volume of 20 $\mu$L/well with 2 $\mu$L of anti-Smadl d2 antibody and 2 $\mu$L of anti-Phospho-Smadl Europium/Terbium cryptate antibody mixture prepared in the detection buffer. Non-treated cell lysate, negative control, control lysate and blank were made according manufacturer's instructions. The plate was then read in time-resolved fluorescence mode, and the homogeneous time-resolved fluorescence (HTRF) signal ratio was determined according to the formula Ratio HTRF = 10.000 $\times$ (Em665nm/Em620nm). Data were analyzed using GraphPad Prism 8 software and statistics p values were determined using the ordinary one-way ANOVA test.

Figure 3A shows that no stimulation of Smad1 phosphorylation occurred in COV434-WT coherent with no expression of AMHR-II receptor (or very low depending the subculture passage). COV434 cell lines express the BMPR2 receptor which is coupled to the Smad pathway. BMP2 -a ligand for BMPR2 receptor- was used as a positive control to verify the functionality of Smad pathway in COV434 cell lines.

Figure 3B shows the specific effect of AMH on COV434 transfected cell line expressing the human AMHR-II receptor. Contrary to Figure 3A, a dose response of Smad1 phosphorylation was achieved with increased AMH concentration at 1ng/mL, 10ng/mL, 100ng/mL and 300ng/mL. 100ng/mL was the concentration that gave a maxima of Smad1 phosphorylation.

Figure 4 shows the percentage of Smad1 phosphorylation obtained with AMH at 100ng/ml, and the competitive effect of the antibodies 4A3 and GM102 at 100$\mu$g/ml. GM102 has no inhibitory effect on Smad1 phosphorylation.

Figure 5 shows the effect of the presence of an antibody according to the invention, 4A3, on the Smad1 phosphorylation induced by AMH. A dose response effect of 4A3 is observed on said phosphorylation, whether AMH is present at a concentration of 1 ng/mL, 10 ng/mL or 100ng/mL. Figure 5A shows the ratio HTRF obtained with AMH at 1ng/ml, 10ng/ml and 100ng/ml and the competitive effect of antibody 4A3 at concentrations 1$\mu$g/ml, 10$\mu$g/ml and 100$\mu$g/ml at each AMH concentration. Figure 5B, 5C, 5D show the corresponding percentage of Smad1 phosphorylation depending the concentration of AMH and the concentration of 4A3 antagonist antibody used.

These results illustrate the fact that an antibody of the invention does not only have a competitive ability, but also an antagonist property when binding to AMHR-II.

## Example 4: ANTIBODY 4A3 INHIBITS IN A DOSE-DEPENDENT MANNER THE SURVIVAL OF TUMOR CELLS

Cell viability assay was assessed using standard MTT assay according to the manufacter's recommendations.

SW620 colorectal cancer cell lines were plated at 15000 cells per well onto 96-well plate. After 24 hours at 37°C under 5% $CO_2$, cells were incubated with serially diluted 4A3 antibody concentrations in quadruplate with or without AMH at 50ng/ml (R&D Systems). Plates were incubated at 37°C under 5% $CO_2$ for a further 48 hours. MTT reagent that enable quantification of viable cells by measurement of metabolic activity was added and plates were read at Optical Density 590nm. Data were analysed using GraphPad Prism 8 software and statistics p values were determined using the ordinary one-way ANOVA test.

Figures 6A and B show the dose-dependant inhibition of SW620 cell viability by 4A3 antibody in presence of AMH at 50ng/ml.

These Figures illustrate a dose-dependent inhibition of the survival of the tumor cells from an antibody of the invention.

**Claims**

1. An antagonist antibody, or a fragment thereof, that specifically binds to the extracellular domain of AMHR-II, in particular a competitive antagonist antibody that specifically binds to the extracellular domain of AMHR-II.

2. The antibody, or a fragment thereof, according to claim 1, said antibody comprising:

   (a) a heavy chain wherein the variable domain comprises:

   - a H-CDR1 having a sequence set forth as SEQ ID NO: 3;
   - a H-CDR2 having a sequence set forth as SEQ ID NO: 4; and
   - a H-CDR3 having a sequence set forth as SEQ ID NO: 5; and

   (b) a light chain wherein the variable domain comprises at least a CDR selected from the group consisting of:

   - a L-CDR1 having a sequence set forth as SEQ ID NO: 8;
   - a L-CDR2 having a sequence set forth as SEQ ID NO: 9; and
   - a L-CDR3 having a sequence set forth as SEQ ID NO: 10.

3. The antibody, or a fragment thereof, according to claim 1 or 2 wherein said antibody is non-immunogenic, and is in particular unable to bind to a Fc receptor.

4. The antibody, or a fragment thereof, according to claim 2 or 3, wherein said antibody comprises a heavy chain variable region having at least 85% identity with the sequence set forth as SEQ ID NO: 2:

   - the H-CDR1 having 100% identity with the sequence set forth as SEQ ID NO: 3;
   - the H-CDR2 having 100% identity with the sequence set forth as SEQ ID NO: 4; and
   - the H-CDR3 having 100% identity with the sequence set forth as SEQ ID NO: 5.

5. The antibody, or a fragment thereof, according to claim 4, wherein the heavy chain variable region of said antibody has at least 90%, in particular at least 95% identity, with the sequence set forth as SEQ ID NO: 2.

6. The antibody, or a fragment thereof, according to any one of claims 2 to 5, wherein said antibody comprises a light chain variable region having at least 85% identity with the sequence set forth as SEQ ID NO: 7:

   - the L-CDR1 having 100% identity with the sequence set forth as SEQ ID NO: 8;
   - the L-CDR2 having 100% identity with the sequence set forth as SEQ ID NO: 9; and
   - the L-CDR3 having 100% identity with the sequence set forth as SEQ ID NO: 10.

7. The antibody, or a fragment thereof, according to claim 6, wherein the light chain variable region of said antibody has at least 90%, in particular at least 95% identity, with the sequence set forth as SEQ ID NO: 7.

8. The antibody, or a fragment thereof, according to any one of claims 2 to 7, wherein said antibody comprises a heavy chain having at least 85% identity with the sequence set forth as SEQ ID NO: 1:

   - the H-CDR1 having 100% identity with the sequence set forth as SEQ ID NO: 3;
   - the H-CDR2 having 100% identity with the sequence set forth as SEQ ID NO: 4; and
   - the H-CDR3 having 100% identity with the sequence set forth as SEQ ID NO: 5.

9. The antibody, or a fragment thereof, according to claim 8, wherein the heavy chain of said antibody has at least 90%, in particular at least 95% identity, with the sequence set forth as SEQ ID NO: 1.

10. The antibody, or a fragment thereof, according to any one of claims 2 to 9, wherein said antibody comprises a light chain having at least 85% identity with the sequence set forth as SEQ ID NO: 6:

    - the L-CDR1 having 100% identity with the sequence set forth as SEQ ID NO: 8;
    - the L-CDR2 having 100% identity with the sequence set forth as SEQ ID NO: 9; and
    - the L-CDR3 having 100% identity with the sequence set forth as SEQ ID NO: 10.

11. The antibody, or a fragment thereof, according to claim 10, wherein the light chain of said antibody has at least 90%, in particular at least 95% identity, with the sequence set forth as SEQ ID NO: 6.

12. The antibody, or a fragment thereof, according to any one of the preceding claims, wherein said antibody comprises a heavy chain having the sequence set forth as SEQ ID NO: 1 and a light chain having the sequence set forth as SEQ ID NO: 6.

13. The antibody, or a fragment thereof, according to claim 1, wherein said antibody, or fragment thereof, competes for binding to the extracellular domain of AMHR-II with the antibody defined in anyone of claims 1 to 12.

14. The antibody, or a fragment thereof, according to claim 1, wherein said antibody, or fragment thereof, competes for binding to the extracellular domain of AMHR-II with AMH.

15. The antibody, or a fragment thereof, according to any one of the preceding claims, wherein said antibody's fragment is selected from the group consisting of Fv, Fab, F(ab')2, Fab', dsFv, scFv, sc(Fv)2 and diabodies.

16. A nucleic acid sequence encoding an antibody, or a fragment thereof, as defined in anyone of claims 1 to 15.

17. A vector comprising a nucleic acid sequence according to claim 16.

18. A host cell comprising a nucleic acid sequence according to claim 16 or a vector according to claim 17.

19. An immunoconjugate comprising the antibody, or a fragment thereof, according to anyone of claims 1 to 15 linked to a therapeutic agent.

20. A pharmaceutical composition comprising, in a pharmaceutically acceptable medium, at least one antibody, or a fragment thereof, as defined in any one of claims 1 to 15 or at least one immunoconjugate as defined in claim 19.

21. An antibody, or a fragment thereof, as defined in any one claim 1 to 15, a cell as defined in claim 18, an immunoconjugate as defined in claim 19 or a composition as defined in claim 20, for its use as a drug, in particular for its use in the prevention and/or treatment of a disease arising from the interaction between AMHR-II and the Anti-Müllerian Hormone (AMH).

22. The antibody, or a fragment thereof, cell or composition for its use according to claim 21, wherein the said disease is selected from the group consisting of:

 - Polycystic ovary syndrome (PCOS);
 - Endometriosis;
 - premature menopause; and
 - cancers associated with the expression of AMHR-II, in particular selected from gynecologic cancers; colon cancer; lung cancer; hepatocellular carcinoma; testis cancer; pancreatic cancer; kidney cancer; breast cancer; thyroid cancer; gastric cancer; adrenal cancer; bladder cancer and prostate cancer.

23. The antibody, or a fragment thereof, cell, immunoconjugate or composition for its use according to any one of claims 21 to 22, in combination with another treatment, in particular another anti-cancer treatment.

**FIGURE 1**

**FIGURE 2**

**FIGURE 3**

**FIGURE 4**

**FIGURE 5**

Sw620_48h_w/o AMH

SW620_48h_with AMH (50ng/ml)

**FIGURE 6**

Europäisches
Patentamt

European
Patent Office

Office européen
des brevets

# EUROPEAN SEARCH REPORT

**Application Number**

EP 19 30 6377

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/025458 A1 (GAMAMABS PHARMA [FR]) 16 February 2017 (2017-02-16) * The whole document, in particular, the examples; figure 6; example 6 * | 1-23 | INV. A61P35/00 A61P43/00 C07K16/28 |
| X,D | WO 2018/189379 A1 (GAMAMABS PHARMA [FR]; INST CURIE [FR]) 18 October 2018 (2018-10-18) * The whole document, in particular the examples and ADCs * | 1-23 | |
| X | WO 2011/045202 A1 (INST NAT SANTE RECH MED [FR]; DI CLEMENTE NATHALIE [FR] ET AL.) 21 April 2011 (2011-04-21) * The whole document, in particular the examples * | 1-23 | |
| X | EP 1 918 304 A1 (INST NAT SANTE RECH MED [FR]) 7 May 2008 (2008-05-07) * The whole document, in particular, the examples * | 1-23 | TECHNICAL FIELDS SEARCHED (IPC) A61K A61P C07K |

The present search report has been drawn up for all claims

| Place of search | Date of completion of the search | Examiner |
|---|---|---|
| Munich | 9 April 2020 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 30 6377

09-04-2020

| Patent document cited in search report | | Publication date | Patent family member(s) | | Publication date |
|---|---|---|---|---|---|
| WO 2017025458 | A1 | 16-02-2017 | EP | 3331569 A1 | 13-06-2018 |
| | | | US | 2017035903 A1 | 09-02-2017 |
| | | | WO | 2017025458 A1 | 16-02-2017 |
| WO 2018189379 | A1 | 18-10-2018 | CA | 3058282 A1 | 18-10-2018 |
| | | | CN | 110891970 A | 17-03-2020 |
| | | | EP | 3609918 A1 | 19-02-2020 |
| | | | KR | 20200014276 A | 10-02-2020 |
| | | | US | 2019367625 A1 | 05-12-2019 |
| | | | WO | 2018189379 A1 | 18-10-2018 |
| WO 2011045202 | A1 | 21-04-2011 | NONE | | |
| EP 1918304 | A1 | 07-05-2008 | AT | 502052 T | 15-04-2011 |
| | | | CA | 2667970 A1 | 08-05-2008 |
| | | | CN | 101573382 A | 04-11-2009 |
| | | | CN | 103396491 A | 20-11-2013 |
| | | | DK | 2097453 T3 | 27-06-2011 |
| | | | EP | 1918304 A1 | 07-05-2008 |
| | | | EP | 2097453 A2 | 09-09-2009 |
| | | | ES | 2363732 T3 | 12-08-2011 |
| | | | JP | 5903717 B2 | 13-04-2016 |
| | | | JP | 2010508810 A | 25-03-2010 |
| | | | JP | 2014076992 A | 01-05-2014 |
| | | | JP | 2016102109 A | 02-06-2016 |
| | | | KR | 20090101897 A | 29-09-2009 |
| | | | KR | 20150036812 A | 07-04-2015 |
| | | | PL | 2097453 T3 | 30-09-2011 |
| | | | US | 2010135996 A1 | 03-06-2010 |
| | | | US | 2013164300 A1 | 27-06-2013 |
| | | | WO | 2008053330 A2 | 08-05-2008 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 404097 A
- WO 9311161 A
- US 4816567 A
- EP 239400 A
- WO 9109967 A
- US 5225539 A
- US 5530101 A
- US 5585089 A
- EP 592106 A
- EP 519596 A
- US 5565332 A
- EP 125023 A
- WO 9602576 A
- US 5545807 A
- US 5545806 A
- US 5569825 A
- US 5625126 A
- US 5633425 A
- US 5661016 A
- US 5750373 A
- WO 2018189379 A
- WO 9514785 A
- WO 9622378 A
- US 5882877 A
- US 6013516 A
- US 4861719 A
- US 5278056 A
- WO 9419478 A
- WO 2017025458 A
- US 20030083263 A
- WO 2002088172 A
- WO 2004010957 A
- US 6884869 B
- US 6323315 B
- US 6239104 B
- US 6034065 A
- US 5780588 A
- US 5665860 A
- US 5663149 A
- US 5635483 A
- US 5599902 A
- US 5554725 A
- US 5530097 A
- US 5521284 A
- US 5504191 A
- US 5410024 A
- US 5138036 A
- US 5076973 A
- US 4986988 A
- US 4978744 A
- US 4879278 A
- US 4816444 A
- US 4486414 A
- US 6130237 A
- WO 8912624 A
- US 6214345 B
- US 5122368 A
- US 5824805 A
- US 5622929 A
- US 4880935 A

### Non-patent literature cited in the description

- **JOSSO N et al.** *Pediatr Endocrinol. Rev.,* 2006, vol. 3 (4), 347-358
- **VISSER et al.** *Reproduction,* 2006, vol. 131 (1), 1-9
- **BAARENDS et al.** *Endocrinology,* 1995, vol. 136 (11), 4951-4962
- **DEWAILLY D et al.** *Hum Reprod Update,* 2014, vol. 20 (3), 370-385
- **PIGNY P et al.** *J Clin Endocrinol Metab,* 2003, vol. 88 (12), 5957-5962
- **CATTEAU-JONARD S et al.** *J Clin Endocrinol Metab,* 2008, vol. 93 (11), 4456-4461
- **DUMONT A et al.** *Reprod Biol Endocrinol,* 2015, vol. 13, 137-146
- **CIMINO I et al.** *Nat. Commun.,* 2016, vol. 7, 10055-10066
- **DEWAILLY D.** *Best Pract Res Clin Obstet Gynaecol,* 2016, vol. 37, 5-11
- **DEWAILLY D et al.** *Hum Reprod,* 2011, vol. 26 (11), 3123-3129
- **PIOUKA et al.** *Am J Physiol Endocrinol Metab,* 2009, vol. 296 (2), E238-E243
- **PELLAT L et al.** *Fertil Steril,* 2011, vol. 92 (1), 240-245
- **SIGNORILE PG et al.** *J Exp Clin Cancer Res,* 2014, vol. 33 (1), 46-54
- **KIM SY et al.** *Obstet Gynecol Sci,* 2018, vol. 61 (1), 127-134

- **VOGELSTEIN et al.** *Science,* 2013, vol. 339 (6127), 1545-1558
- **LEVY et al.** *Molec Cell biol,* 2005, vol. 25 (18), 8108-8125
- **MORIKAWA et al.** *Cold Spring Harb. Perspect.,* 2016, vol. 8 (5), a021873
- **LUO et al.** *Transl. Oncol.,* 2019, vol. 12 (3), 475-484
- **FURLER et al.** *Cancers,* 2018, vol. 10 (6), E199
- **BAKKUM-GAMEZ JN et al.** *Gynecol. Oncol.,* 2008, vol. 108 (1), 141-148
- **KIM SM et al.** *Oncol Letter,* 2019, vol. 17 (1), 532-538
- **TAXIMAIMAITA R et al.** *Medicine,* 2018, vol. 97 (22), e10793
- **M. GOWKIELEWICZ et al.** *Int. J. Mol. Sci.,* 2019, vol. 20 (6), 1325
- **BARRET et al.** *Proc Amer Soc Clin Oncol,* 2018, vol. 37
- **IMBEAUD et al.** *Nature Genet,* 1995, vol. 11, 382-388
- The Pharmacology of Monoclonal Antibodies. Springer- Verlag, 1994, vol. 113, 269-315
- **HOLLINGER et al.** *Proc. Natl. Acad. Sci. USA,* 1993, vol. 90, 6444-6448
- **SPIESS et al.** *Mol Immunol.,* 2015, vol. 67 (2), 95-106
- **KONTERMANN et al.** Bispecific antibodies. *Drug Discov Today,* 2015, vol. 20 (7), 838-47
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VAUGHAN et al.** *Nature Biotechnology,* 1996, vol. 14, 309-314
- **SHEETS et al.** *Proc. Natl. Acad. Sci.,* 1998, vol. 95, 6157-6162
- **HOOGENBOOM ; WINTER.** *J. Mol. Biol,* 1991, vol. 227, 381
- **MARKS et al.** *J. Mol. Biol,* 1991, vol. 222, 581
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **COLE et al.** Monoclonal Antibodies and Cancer Therapy. Alan R. Liss, 1985, 77
- **BOERNER et al.** *J. Immunol,* 1991, vol. 147 (1), 86-95
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **E. MEYERS ; W. MILLER.** *Comput. Appl. Biosci.,* 1988, vol. 4 (1), 1-17
- **NEEDLEMAN ; WUNSCH.** *J. Mol, Biol.,* 1970, vol. 48, 444-453
- **YAMANE-OHNUKI et al.** *Biotechnol Bioeng,* 2004, vol. 87 (5), 614-622
- **SATOH et al.** *Expert Opin Biol Ther,* 2006, vol. 6 (11), 1161-1173
- **LI et al.** *Cancer Res.,* 1982, vol. 42, 999-1004
- **CHARI et al.** *Cancer Res.,* 1992, vol. 52, 127-131
- Monoclonal Antibodies For Immunotargeting Of Drugs In Cancer Therapy. **ARNON et al.** Monoclonal Antibodies And Cancer Therapy. Alan R. Liss, Inc, 1985
- Antibodies For Drug Delivery. **HELLSTROM et al.** Controlled Drug Delivery. Marcel Deiker, Inc, 1987
- Antibody Carriers Of Cytotoxic Agents In Cancer Therapy: A Review. Monoclonal Antibodies '84: Biological And Clinical Applications. 1985
- Analysis, Results, and Future Prospective of the Therapeutic Use of Radiolabeled Antibody In Cancer Therapy. Monoclonal Antibodies For Cancer Detection And Therapy. Academic Press, 1985
- **THORPE et al.** *Immunol. Rev.,* 1982, vol. 62, 119-58
- **DUBOWCHIK ; WALKER.** *Pharm. Therapeutics,* 1999, vol. 83, 67-123
- **NEVILLE et al.** *Biol. Chem.,* 1989, vol. 264, 14653-14661
- **THORPE et al.** *Cancer Res.,* 1987, vol. 47, 5924-5931
- **WAWRZYNCZAK et al.** Immunoconjugates: Antibody Conjugates in Radioimagery and Therapy of Cancer. Oxford U. Press, 1987
- **JOHNSON et al.** *Anticancer Res.,* 1995, vol. 15, 1387-93
- **LAU et al.** *Bioorg-Med-Chem.,* 1995, vol. 3 (10), 1299-1304
- **LAU et al.** *Bioorg-Med-Chem.,* 1995, vol. 3 (10), 1305-12
- Remington's Pharmaceutical Sciences. Mack Publishing Company, 1995
- Remington's Pharmaceutical Sciences. 1035-1038, 1570-1580
- **PIERRE A et al.** *J Clin Endocrin Metab,* 2017, vol. 102 (11), 3970-3978
- **SIGNORILE et al.** *J Exp Clin Cancer,* 2014, vol. 33 (46), 1-9

- **VICTORIA et al.** *J Gynecol Hum Reprod,* 2019, vol. 48, 19-24
- **BARRET et al.** *Proc AACR Annual Meeting,* 2018